# EUROPÄISCHE PATENTSCHRIFT

(11) **EP 1 189 893 B1**
(45) Veröffentlichungstag und Bekanntmachung des Hinweises auf die Patenterteilung: **07.09.2005**
(21) Anmeldenummer: 00940265.2
(22) Anmeldetag: 24.05.2000
(51) Int. Cl.: C07D 253/06, C07D 409/12, C07D 401/12, C07D 405/12, C07D 417/12, C07D 413/12, A01N 43/653

(54) **SUBSTITUIERTE 2-ARYL-1,2,4-TRIAZIN-3,5-DI(THIO)ONE ALS HERBIZIDE**
SUBSTITUTED 2-ARYL-1,2,4-TRIAZINE-3,5-DI(THI)ONE
2-ARYL-1,2,4-TRIAZIN-3,5-DI(THI)ONES SUBSTITUEES

(30) Priorität: 04.06.1999 DE 19925593
(43) Veröffentlichungstag der Anmeldung: 27.03.2002
(73) Patentinhaber: Bayer CropScience AG, 40789 Monheim (DE)
(72) Erfinder: LINKER, Karl-Heinz, D-51377 Leverkusen (DE); KLUTH, Joachim, D-40764 Langenfeld (DE); DREWES, Mark, Wilhelm, D-40764 Langenfeld (DE); DAHMEN, Peter, D-41470 Neuss (DE); FEUCHT, Dieter, D-40789 Monheim (DE); PONTZEN, Rolf, D-42799 Leichlingen (DE)
(86) Internationale Anmeldenummer: PCT/EP2000/004704
(87) Internationale Veröffentlichungsnummer: WO 2000/075119

(56) Entgegenhaltungen:
- WO-A-97/30980
- WO-A-99/05125
- DE-A- 19 516 785

## Beschreibung

Die Erfindung betrifft neue substituierte 2-Aryl-1,2,4-triazin-3,5-di(thi)one, Verfahren zu ihrer Herstellung und ihre Verwendung als Herbizide.

Es ist bekannt, dass bestimmte substituierte 2-Aryl-1,2,4-triazin-(thi)one herbizide Eigenschaften aufweisen (vgl. EP-A-0 011 693, EP-A-0 271 170, WO-A-86/00072, WO-A-97/30980, US-A-4,755,217, US-A-4,878,941, US-A-4,956,004, US-A-5,262,390, US-A-5,344,812). In WO-A-99/05125 werden ebenfalls bereits herbizid wirksame 2-Aryl-1,2,4-triazin-3,5-di(thi)one beschrieben. Die aus den angegebenen Patentanmeldungen bzw. Patenten bekannten Verbindungen haben jedoch keine nennenswerte Bedeutung erlangt.

Es wurden nun neue substituierte 2-Aryl-1,2,4-triazin-3,5-di(thi)one der allgemeinen Formel (I) 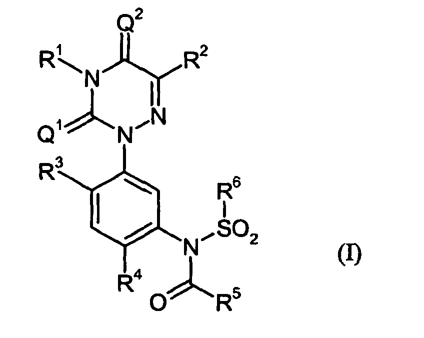 in welcher
- Q¹: für Sauerstoff oder Schwefel steht,
- Q²: für Sauerstoff oder Schwefel steht,
- R¹: für Wasserstoff, Cyano, Amino, für jeweils gegebenenfalls durch Cyano, Halogen oder C₁-C₄-Alkoxy substituiertes Alkyl, Alkoxy, Alkylamino, Di-alkylamino, Alkylcarbonyl oder Alkoxycarbonyl mit jeweils 1 bis 6 Kohlenstoffatomen in den Alkylgruppen, für jeweils gegebenenfalls durch Halogen substituiertes Alkenyl, Alkenylcarbonyl, Alkenyloxycarbonyl, Alkinyl, Alkinylcarbonyl oder Alkinyloxycarbonyl mit jeweils 2 bis 6 Kohlenstoffatomen in den Alkenyl- bzw. Alkinyl-gruppen, oder für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil steht,
- R²: für Wasserstoff, Halogen, Nitro, Carboxy, Cyano, Thiocarbamoyl, Amino, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen, für jeweils gegebenenfalls durch Halogen substituiertes Alkenyl, Alkenyloxy, Alkenylthio, Alkinyl, Alkinyloxy oder Alkinylthio mit jeweils 2 bis 6 Kohlenstoffatomen in den Alkenyl- oder Alkinyl-gruppen, oder für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 6 Kohlenstoffatomen im Alkylteil steht,
- R³: für Wasserstoff, Cyano oder Halogen steht,
- R⁴: für Cyano oder Thiocarbamoyl steht,
- R⁵: für Wasserstoff, für Alkoxycarbonyl mit 1 bis 6 Kohlenstoffatomen in der Alkoxygruppe, oder für einen der Reste -R⁷, -O-R⁷, -SR⁷, -NH-R⁷ oder - NR⁷R⁸ steht,
- R⁶: für Amino, Hydroxy oder für einen der Reste -R⁷ oder -NR⁷R⁸ steht,
- R⁷: für gegebenenfalls einfach oder mehrfach, gleich oder verschieden substituiertes, geradkettiges oder verzweigtes Alkyl mit 1 bis 10 Kohlenstoffatomen steht, wobei als Substituenten in Betracht kommen: Cyano, Carboxy, Carbamoyl, Thiocarbamoyl, Halogen, jeweils geradkettiges oder verzweigtes Alkoxy, Alkoxyalkoxy, Alkylcarbonyloxy, Alkylthio, Alkylsulfinyl, Alkylsulfonyl, Alkoxycarbonyl, N-Alkyl-aminocarbonyl, N,N-Dialkyl-aminocarbonyl, Trialkylsilyl oder Alkylsulfonylaminocarbonyl mit jeweils 1 bis 6 Kohlenstoffatomen in den einzelnen Alkylteilen, oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht, oder
- R⁷: für jeweils gegebenenfalls einfach oder mehrfach, gleich oder verschieden durch Halogen substituiertes Alkenyl oder Alkinyl mit jeweils 2 bis 8 Kohlenstoffatomen steht, oder
- R⁷: für jeweils gegebenenfalls einfach oder mehrfach, gleich oder verschieden durch Cyano, Halogen und/oder C₁-C₄-Alkyl substituiertes Cycloalkyl oder Cycloalkylalkyl mit jeweils 3 bis 7 Kohlenstoffatomen in den Cycloalkylgruppen und gegebenenfalls 1 bis 4 Kohlenstoffatomen im Alkylteil steht, oder
- R⁷: für jeweils gegebenenfalls im Arylteil einfach oder mehrfach, gleich oder verschieden substituiertes Aryl, Arylalkyl, Bis-aryl-alkyl, Arylalkenyl, Aryloxyalkyl oder Arylalkoxyalkyl mit jeweils 6 bis 10 Kohlenstoffatomen im Arylteil und gegebenenfalls bis zu 4 Kohlenstoffatomen im geradkettigen oder verzweigten Alkylteil bzw. Alkenylteil steht, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl-bzw. Heterocyclylsubstituenten in Betracht kommen:
Halogen, Cyano, Nitro, Amino, Methylendioxy, N-(C₁-C₄-Alkyl-carbonyl)amino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 3 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 3 gleichen oder verschiedenen Halogenatomen substituiertes Phenyl, und
- R⁸: für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten in Betracht kommen:
Cyano, Carboxy, Carbamoyl, Thiocarbamoyl, Halogen, jeweils geradkettiges oder verzweigtes Alkoxy, Alkoxyalkoxy, Alkylthio, Alkylsulfinyl, Alkylsulfonyl, Alkoxycarbonyl, Alkylaminocarbonyl, Dialkylaminocarbonyl, Tri-alkylsilyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/ oder Schwefel - steht, oder
- R⁸: für jeweils gegebenenfalls einfach oder mehrfach, gleich oder verschieden durch Halogen substituiertes Alkenyl oder Alkinyl mit jeweils 2 bis 8 Kohlenstoffatomen steht, oder
- R⁸: für gegebenenfalls einfach oder mehrfach, gleich oder verschieden durch Cyano, Halogen und/oder geradkettiges oder verzweigtes Alkyl mit 1 bis 4 Kohlenstoffatomen substituiertes Cycloalkyl mit 3 bis 7 Kohlenstoffatomen steht, oder
- R⁸: mit R⁷ für gegebenenfalls durch O (Sauerstoff), S (Schwefel), NH oder N(C₁-C₄-Alkyl) unterbrochenes Alkandiyl (Alkylen) mit 2 bis 6 Kohlenstoffatomen steht,
gefunden.

Bevorzugte Substituenten bzw. Bereiche der in den oben und nachstehend aufgeführten Formeln vorhandenen Reste werden im folgenden definiert.
- R¹: steht bevorzugt für Wasserstoff, Cyano, Amino, für jeweils gegebenenfalls durch Cyano, Fluor, Chlor, Methoxy oder Ethoxy substituiertes Methyl, Ethyl, n- oder i-Propyl, n-, i- s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino, Dimethylamino, Diethylamino, Acetyl, Propionyl, n- oder i-Butyroyl, Methoxycarbonyl, Ethoxycarbonyl, n- oder i-Propoxycarbonyl, für jeweils gegebenenfalls durch Fluor, Chlor oder Brom substituiertes Propenyl, Butenyl, Propenylcarbonyl, Butenylcarbonyl, Propenyloxycarbonyl, Butenyloxycarbonyl, für Propinyl, Butinyl, Propinylcarbonyl, Butinylcarbonyl, Propinyloxycarbonyl oder Butinyloxycarbonyl, oder für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl.
- R²: steht bevorzugt für Wasserstoff, Nitro, Carboxy, Cyano, Thiocarbamoyl, Amino, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino, für jeweils gegebenenfalls durch Fluor, Chlor oder Brom substituiertes Propenyl, Propenyloxy, Propenylthio, Butenyl, Butenyloxy oder Butenylthio, für Propinyl, Propinyloxy, Propinylthio, Butinyl, Butinyloxy oder Butinylthio, oder für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl.
- R³: steht bevorzugt für Wasserstoff, Fluor oder Chlor.
- R⁵: steht bevorzugt für Wasserstoff, Methoxycarbonyl, Ethoxycarbonyl, n- oder i-Propoxycarbonyl, oder für einen der Reste -R⁷, -O-R⁷, -S-R⁷, -NH-R⁷ oder -NR⁷R⁸.
- R⁷: steht bevorzugt für jeweils gegebenenfalls einfach oder zweifach substituiertes Methyl, Ethyl, n- oder i-Propyl, n-, i-, s- oder t-Butyl, n-, i-, s-, t-oder neo-Pentyl, n-, i- oder s-Hexyl, wobei als Substituenten jeweils vorzugsweise in Betracht kommen:
Cyano, Carboxy, Carbamoyl, Fluor, Chlor, Brom, Methoxy, Ethoxy, n- oder i-Propoxy, Methoxymethoxy, Ethoxymethoxy, Methoxyethoxy, Ethoxyethoxy, Acetylmethoxy, Methylthio, Ethylthio, n- oder i-Propylthio, Methylsulfinyl, Ethylsulfinyl, Methylsulfonyl, Ethylsulfonyl, Methoxycarbonyl, Ethoxycarbonyl, Methylaminocarbonyl, Ethyl-aminocarbonyl, Dimethylaminocarbonyl, Trimethylsilyl, Methylsulfonylaminocarbonyl oder Ethylsulfonylaminocarbonyl.
- R⁷: steht weiterhin bevorzugt für jeweils gegebenenfalls einfach oder zweifach durch Fluor und/oder Chlor substituiertes Ethenyl, Propenyl, Butenyl, Ethinyl, Propinyl oder Butinyl.
- R⁷: steht weiterhin bevorzugt für jeweils gegebenenfalls einfach oder zweifach, gleich oder verschieden durch Fluor, Chlor, Methyl und/oder Ethyl substituiertes Cyclopropyl, Cyclobutyl, Cyclopentyl, Cyclohexyl, Cyclopropylmethyl, Cyclopropylethyl, Cyclopentylmethyl, Cyclopentylethyl, Cyclohexylmethyl oder Cyclohexylethyl.
- R⁷: steht weiterhin bevorzugt für jeweils gegebenenfalls einfach, zweifach oder dreifach, gleich oder verschieden substituiertes Phenyl, Benzyl, Phenylethyl, Phenylpropyl, 2,2-Bis-phenyl-ethyl, Phenylethenyl, Phenoxymethyl, Phenoxyethyl, Phenoxypropyl, Furyl, Furylmethyl, Thienyl, Oxazolyl, Isoxazolyl, Thiazolyl, Pyrazolyl, Oxadiazolyl, Thiadiazolyl, Pyridinyl, Pyridinylmethyl, Pyrimidinyl, Pyrrolidinyl, Piperidinyl, Morpholinyl oder Chromanyl, wobei als Substituenten jeweils vorzugsweise in Betracht kommen:
Fluor, Chlor, Brom, Cyano, Nitro, Amino, Methylendioxy, N-Acetyl-amino, Methyl, Ethyl, n- oder i-Propyl, n-, i-, s- oder t-Butyl, Methoxy, Ethoxy, n-oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl, Methoxycarbonyl, Ethoxycarbonyl, Methoximinomethyl, Methoximinoethyl, Ethoximinomethyl, Ethoximinoethyl.
- R⁸: steht bevorzugt für Wasserstoff oder für jeweils gegebenenfalls einfach substituiertes Methyl, Ethyl, n- oder i-Propyl, n- oder i-Butyl, wobei als Substituenten vorzugsweise in Betracht kommen:
Cyano, Carboxyl, Carbamoyl, Fluor, Chlor, Methoxy, Ethoxy, n- oder i-Propoxy, Methoxymethoxy, Ethoxymethoxy, Methoxyethoxy, Ethoxyethoxy, Methylthio, Ethylthio, n- oder i-Propylthio, Methylsulfinyl, Ethylsulfinyl, Methylsulfonyl, Ethylsulfonyl, Methoxycarbonyl, Ethoxycarbonyl, Methylaminocarbonyl, Ethylaminocarbonyl, Dimethylaminocarbonyl.
- R⁸: steht weiterhin bevorzugt für jeweils gegebenenfalls einfach oder zweifach durch Fluor und/oder Chlor - substituiertes Ethenyl, Propenyl, Butenyl, Ethinyl, Propinyl oder Butinyl.
- R⁸: steht weiterhin bevorzugt für gegebenenfalls einfach oder zweifach, gleich oder verschieden durch Fluor, Chlor, Methyl und/oder Ethyl substituiertes Cyclopropyl, Cyclopentyl oder Cyclohexyl.
- R⁸: steht weiterhin bevorzugt zusammen mit R⁷ für Butan-1,4-diyl, Pentan-1,5-diyl oder 3-Oxa-pentan-1,5-diyl.
- R¹: steht besonders bevorzugt für Wasserstoff oder für jeweils gegebenenfalls durch Fluor substituiertes Methyl, Ethyl, n- oder i-Propyl.
- R²: steht besonders bevorzugt für Wasserstoff, Cyano, Fluor, Chlor, Brom oder für jeweils gegebenenfalls durch Fluor substituiertes Methyl, Ethyl, n- oder i-Propyl.
- R⁵: steht besonders bevorzugt für Wasserstoff oder für einen der Reste -R⁷, -O-R⁷, -S-R⁷, -NH-R⁷ oder -NR⁷R⁸.
- R⁷: steht besonders bevorzugt für jeweils gegebenenfalls einfach oder zweifach substituiertes Methyl, Ethyl, n- oder i-Propyl, n-, i-, s- oder t-Butyl, n-, i-, s-, t- oder neo-Pentyl, wobei als Substituenten jeweils vorzugsweise in Betracht kommen:
Cyano, Fluor, Chlor, Brom, Methoxy, Ethoxy, n- oder i-Propoxy, Methoxymethoxy, Ethoxymethoxy, Methoxyethoxy, Ethoxyethoxy, Acetyloxymethyl, Acetylmethoxy.
- R⁷: steht weiterhin besonders bevorzugt für jeweils gegebenenfalls einfach oder zweifach durch Fluor und/oder Chlor substituiertes Ethenyl, Propenyl, Butenyl, Ethinyl, Propinyl oder Butinyl.
- R⁷: steht weiterhin besonders bevorzugt für jeweils gegebenenfalls einfach oder zweifach, gleich oder verschieden durch Fluor, Chlor, Methyl und/oder Ethyl substituiertes Cyclopropyl, Cyclobutyl, Cyclopentyl, Cyclohexyl, Cyclopropylmethyl, Cyclopropylethyl, Cyclopentylmethyl, Cyclopentylethyl, Cyclohexylmethyl oder Cyclohexylethyl.
- R⁷: steht weiterhin besonders bevorzugt für jeweils gegebenenfalls einfach, zweifach oder dreifach, gleich oder verschieden substituiertes Phenyl, Benzyl, Phenylethyl, Phenylpropyl, 2,2-Bis-phenyl-ethyl, Phenylethenyl, Phenoxymethyl, Phenoxyethyl, Phenoxypropyl, Furyl, Thienyl, Oxazolyl, Isoxazolyl, Thiazolyl, Pyrazolyl, Oxadiazolyl, Thiadiazolyl, Pyridinyl, Pyrimidinyl, Pyrrolidinyl, Piperidinyl oder Chromanyl, wobei als Substituenten jeweils vorzugsweise in Betracht kommen:
Fluor, Chlor, Brom, Cyano, Nitro, Methylendioxy, Methyl, Ethyl, n- oder i-Propyl, n-, i-, s- oder t-Butyl, Methoxy, Ethoxy, n- oder i-Propoxy, Methylthio, Ethylthio, Methylsulfinyl, Methylsulfonyl, Trifluormethyl, Difluormethyl, Trifluormethoxy, Difluormethoxy.
- R⁸: steht besonders bevorzugt für Wasserstoff oder für jeweils gegebenenfalls einfach substituiertes Methyl, Ethyl, n- oder i-Propyl, n- oder i-Butyl, wobei als Substituenten vorzugsweise in Betracht kommen:
Cyano, Fluor, Chlor, Methoxy, Ethoxy, n- oder i-Propoxy.
- R⁸: steht weiterhin besonders bevorzugt für jeweils gegebenenfalls einfach oder zweifach durch Fluor und/oder Chlor - substituiertes Ethenyl, Propenyl, Butenyl, Ethinyl, Propinyl oder Butinyl.
- R⁸: steht weiterhin besonders bevorzugt für gegebenenfalls einfach oder zweifach, gleich oder verschieden durch Fluor, Chlor, Methyl und/oder Ethyl substituiertes Cyclopropyl, Cyclopentyl oder Cyclohexyl.
- R¹: steht insbesondere bevorzugt für Methyl, Ethyl oder n- oder i-Propyl.
- R²: steht insbesondere bevorzugt für Wasserstoff.
- R³: steht insbesondere bevorzugt für Fluor oder Chlor.
- R⁶: steht insbesondere bevorzugt für Methyl, Ethyl oder n- oder i-Propyl.

Die oben aufgeführten allgemeinen oder in Vorzugsbereichen aufgeführten Restedefinitionen gelten sowohl für die Endprodukte der Formel (I) als auch entsprechend für die jeweils zur Herstellung benötigten Ausgangs- oder Zwischenprodukte. Diese Restedefinitionen können untereinander, also auch zwischen den angegebenen bevorzugten Bereichen beliebig kombiniert werden.

Erfindungsgemäß bevorzugt sind diejenigen Verbindungen der Formel (I), bei welchen eine Kombination der vorstehend als bevorzugt aufgeführten Bedeutungen vorliegt.

Erfindungsgemäß besonders bevorzugt sind diejenigen Verbindungen der Formel (I), bei welchen eine Kombination der vorstehend als besonders bevorzugt aufgeführten Bedeutungen vorliegt.

Erfindungsgemäß insbesondere bevorzugt sind diejenigen Verbindungen der Formel (I), bei welchen eine Kombination der vorstehend als insbesondere bevorzugt aufgeführten Bedeutungen vorliegt.

Gesättigte oder ungesättigte Kohlenwasserstoffreste, wie Alkyl oder Alkenyl, sind - auch in Verbindung mit Heteroatomen, wie in Alkoxy - soweit möglich jeweils geradkettig oder verzweigt.

Gegebenenfalls substituierte Reste können einfach oder mehrfach substituiert sein, wobei bei Mehrfachsubstitution die Substituenten gleich oder verschieden sein können.

Die neuen substituierten 2-Aryl-1,2,4-triazin-3,5-di(thi)one der allgemeinen Formel (I) weisen interessante biologische Eigenschaften auf. Sie zeichnen sich insbesondere durch starke herbizide Wirksamkeit aus.

Man erhält die neuen substituierten 2-Aryl-1,2,4-triazin-3,5-di(thi)one der allgemeinen Formel (I), wenn man 2-Aryl-1,2,4-triazin-3,5-di(thi)one der allgemeinen Formel (II) 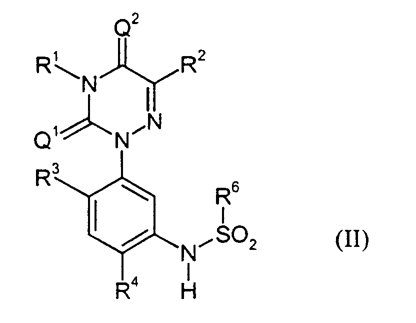 in welcher
Q¹, Q², R¹, R², R³, R⁴ und R⁶ die oben angegebene Bedeutung haben,
mit Halogencarbonylverbindungen der allgemeinen Formel (III) 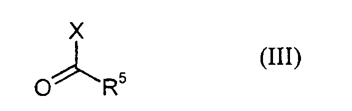 in welcher
- R⁵: die oben angegebene Bedeutung hat und
- X: für Halogen steht,
gegebenenfalls in Gegenwart eines Reaktionshilfsmittels und gegebenenfalls in Gegenwart eines Verdünnungsmittels umsetzt.

Verwendet man beispielsweise 2-(4-Cyano-2-fluor-5-methylsulfonylamino-phenyl)-4-methyl-1,2,4-triazin-3,5(2H,4H)-dion und Propionsäurechlorid als Ausgangsstoffe, so kann der Reaktionsablauf beim erfindungsgemäßen Verfahren durch das folgende Formelschema skizziert werden:

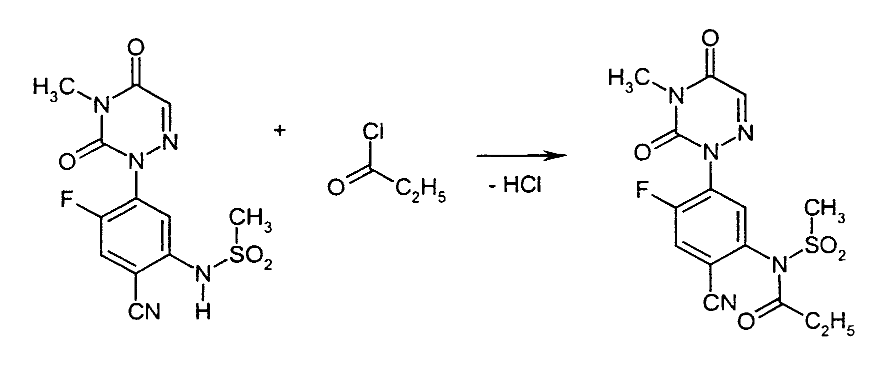

Die beim erfindungsgemäßen Verfahren zur Herstellung von Verbindungen der allgemeinen Formel (I) als Ausgangsstoffe zu verwendenden 2-Aryl-1,2,4-triazin-3,5-di(thi)one sind durch die Formel (II) allgemein definiert. In der allgemeinen Formel (II) haben Q¹, Q², R¹, R², R³, R⁴ und R⁶ vorzugsweise bzw. insbesondere diejenigen Bedeutungen, die bereits oben im Zusammenhang mit der Beschreibung der erfindungsgemäßen Verbindungen der allgemeinen Formel (I) vorzugsweise bzw. als insbesondere bevorzugt für Q¹, Q², R¹, R², R³, R⁴ und R⁶ angegeben worden sind.

Die Ausgangsstoffe der allgemeinen Formel (II) sind bekannt und/oder können nach an sich bekannten Verfahren hergestellt werden (vgl. WO-A-97/30980, Herstellungsbeispiele).

Die beim erfindungsgemäßen Verfahren zur Herstellung von Verbindungen der allgemeinen Formel (I) weiter als Ausgangsstoffe zu verwendenden Halogencarbonylverbindungen sind durch die Formel (III) allgemein definiert. In der allgemeinen Formel (III) hat R⁵ vorzugsweise bzw. insbesondere diejenige Bedeutung, die bereits oben im Zusammenhang mit der Beschreibung der erfindungsgemäßen Verbindungen der allgemeinen Formel (I) vorzugsweise bzw. als insbesondere bevorzugt für R⁵ angegeben worden ist; X steht vorzugsweise für Fluor, Chlor oder Brom, insbesondere für Chlor.

Die Ausgangsstoffe der allgemeinen Formel (III) sind bekannte organische Synthesechemikalien.

Als Reaktionshilfsmittel für das erfindungsgemäßeVerfahren kommen im allgemeinen die üblichen anorganischen oder organischen Basen oder Säureakzeptoren in Betracht. Hierzu gehören vorzugsweise Alkalimetall- oder Erdalkalimetall- -acetate, -amide, -carbonate, -hydrogencarbonate, -hydride, -hydroxide oder -alkanolate, wie beispielsweise Natrium-, Kalium- oder Calcium-acetat, Lithium-, Natrium-, Kalium- oder Calcium-amid, Natrium-, Kalium- oder Calcium-carbonat, Natrium-, Kalium- oder Calcium-hydrogencarbonat, Lithium-, Natrium-, Kalium- oder Calcium-hydrid, Lithium-, Natrium-, Kalium- oder Calcium-hydroxid, Natrium- oder Kalium- -methanolat, -ethanolat, -n- oder -i-propanolat, -n-, -i-, -s- oder -t-butanolat; weiterhin auch basische organische Stickstoffverbindungen, wie beispielsweise Trimethylamin, Triethylamin, Tripropylamin, Tributylamin, Ethyl-diisopropylamin, N,N-Dimethyl-cyclohexylamin, Dicyclohexylamin, Ethyl-dicyclohexylamin, N,N-Dimethyl-anilin, N,N-Dimethyl-benzylamin, Pyridin, 2-Methyl-, 3-Methyl-, 4-Methyl-, 2,4-Dimethyl-, 2,6-Dimethyl-, 3,4-Dimethyl- und 3,5-Dimethyl-pyridin, 5-Ethyl-2-methyl-pyridin, 4-Dimethylamino-pyridin, N-Methyl-piperidin, 1,4-Diazabicyclo-[2,2,2]-octan (DABCO), 1,5-Diazabicyclo[4,3,0]-non-5-en (DBN), oder 1,8 Diazabicyclo[5,4,0]-undec-7-en (DBU).

Als weitere Reaktionshilfsmittel für das erfindungsgemäßen Verfahren kommen auch Phasentransfer-Katalysatoren in Betracht. Als Beispiele für solche Katalysatoren seien genannt:

Tetrabutylammonium-bromid, Tetrabutylammonium-chlorid, Tetraoctylammoniumchlorid, Tetrabutylammonium-hydrogensulfat, Methyl-trioctylammonium-chlorid, Hexadecyl-trimethylammonium-chlorid, Hexadecyl-trimethylammonium-bromid, Benzyl-trimethylammonium-chlorid, Benzyl-triethylammonium-chlorid, Benzyl-trimethylammonium-hydroxid, Benzyl-triethylammonium-hydroxid, Benzyl-tributylammonium-chlorid, Benzyl-tributylammonium-bromid, Tetrabutylphosphoniumbromid, Tetrabutylphosphonium-chlorid, Tributyl-hexadecylphosphonium-bromid, Butyl-triphenylphosphonium-chlorid, Ethyl-trioctylphosphonium-bromid, Tetraphenylphosphonium-bromid.

Als Verdünnungsmittel zur Durchführung des erfindungsgemäßen Verfahrens kommen neben Wasser vor allem inerte organische Lösungsmittel in Betracht. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Butyronitril; Amide, wie N,N-Dimethylformamid, N,N-Dimethylacetamid, N-Methyl-formanilid, N-Methyl-pyrrolidon oder Hexamethylphosphorsäuretriamid; Ester wie Essigsäuremethylester oder Essigsäureethylester, Sulfoxide, wie Dimethylsulfoxid, Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die Reaktionstemperaturen können bei der Durchführung des erfindungsgemäßen Verfahrens in einem größeren Bereich variiert werden. Im allgemeinen arbeitet man bei Temperaturen zwischen 0°C und 150°C, vorzugsweise zwischen 10°C und 120°C.

Das erfindungsgemäße Verfahren wird im allgemeinen unter Normaldruck durchgeführt. Es ist jedoch auch möglich, das erfindungsgemäße Verfahren unter erhöhtem oder vermindertem Druck - im allgemeinen zwischen 0,1 bar und 10 bar - durchzuführen.

Zur Durchführung des erfindungsgemäßen Verfahrens werden die Ausgangsstoffe im allgemeinen in angenähert äquimolaren Mengen eingesetzt. Es ist jedoch auch möglich, eine der Komponenten in einem größeren Überschuss zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:
Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindemia, Lamium, Veronica, Abutilon, Emex, Datura, Viola, Galeopsis, Papaver, Centaurea, Trifolium, Ranunculus, Sphenoclea, Taraxacum.
Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.
Monokotyle Unkräuter der Gattungen: Echinochloa, Eriochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Dactyloctenium, Agrostis, Alopecurus, Apera, Aegilops, Phalaris.
Monokotyle Kulturen der Gattungen: Oryza, Zea, Triticum, Hordeum, Avena, Secale, Sorghum, Panicum, Saccharum, Ananas, Asparagus, Allium.
Die Verwendung der erfindungsgemäßen Wirkstoffe ist jedoch keineswegs auf diese Gattungen beschränkt, sondern erstreckt sich in gleicher Weise auch auf andere Pflanzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Sproß, Blatt, Blüte und Wurzel verstanden werden, wobei beispielhaft Blätter, Nadeln, Stengel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Die erfindungsgemäßen Wirkstoffe eignen sich in Abhängigkeit von der Konzentration zur Totalunkrautbekämpfung, z.B. auf Industrie- und Gleisanlagen und auf Wegen und Plätzen mit und ohne Baumbewuchs. Ebenso können die erfindungsgemäßen Wirkstoffe zur Unkrautbekämpfung in Dauerkulturen, z.B. Forst, Ziergehölz-, Obst-, Wein-, Citrus-, Nuss-, Bananen-, Kaffee-, Tee-, Gummi-, Ölpalm-, Kakao-, Beerenfrucht- und Hopfenanlagen, auf Zier- und Sportrasen und Weideflächen sowie zur selektiven Unkrautbekämpfung in einjährigen Kulturen eingesetzt werden.

Die erfindungsgemäßen Verbindungen der Formel (I) zeigen starke herbizide Wirksamkeit und ein breites Wirkungsspektrum bei Anwendung auf dem Boden und auf oberirdische Pflanzenteile. Sie eignen sich in gewissem Umfang auch zur selektiven Bekämpfung von monokotylen und dikotylen Unkräutern in monokotylen und dikotylen Kulturen, sowohl im Vorauflauf- als auch im Nachauflauf-Verfahren.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan. Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide infrage, beispielsweise
Acetochlor, Acifluorfen(-sodium), Aclonifen, Alachlor, Alloxydim(-sodium), Ametryne, Amidochlor, Amidosulfuron, Anilofos, Asulam, Atrazine, Azafenidin, Azimsulfuron, Benazolin(-ethyl), Benfuresate, Bensulfuron(-methyl), Bentazon, Benzobicyclon, Benzofenap, Benzoylprop(-ethyl), Bialaphos, Bifenox, Bispyribac(sodium), Bromobutide, Bromofenoxim, Bromoxynil, Butachlor, Butroxydim, Butylate, Cafenstrole, Caloxydim, Carbetamide, Carfentrazone(-ethyl), Chlomethoxyfen, Chloramben, Chloridazon, Chlorimuron(-ethyl), Chlornitrofen, Chlorsulfuron, Chlortoluron, Cinidon(-ethyl), Cinmethylin, Cinosulfuron, Clefoxydim, Clethodim, Clodinafop(-propargyl), Clomazone, Clomeprop, Clopyralid, Clopyrasulfuron(-methyl), Cloransulam(-methyl), Cumyluron, Cyanazine, Cybutryne, Cycloate, Cyclosulfamuron, Cycloxydim, Cyhalofop(-butyl), 2,4-D, 2,4-DB, 2,4-DP, Desmedipham, Diallate, Dicamba, Diclofop(-methyl), Diclosulam, Diethatyl(-ethyl), Difenzoquat, Diflufenican, Diflufenzopyr, Dimefuron, Dimepiperate, Dimethachlor, Dimethametryn, Dimethenamid, Dimexyflam, Dinitramine, Diphenamid, Diquat, Dithiopyr, Diuron, Dymron, Epropodan, EPTC, Esprocarb, Ethalfluralin, Ethametsulfuron(-methyl), Ethofumesate, Ethoxyfen, Ethoxysulfuron, Etobenzanid, Fenoxaprop-(-P-ethyl), Fentrazamide, Flamprop(-isopropyl), Flamprop(-isopropyl-L), Flamprop(methyl), Flazasulfuron, Florasulam, Fluazifop(-P-butyl), Fluazolate, Flucarbazone, Flufenacet, Flumetsulam, Flumiclorac(-pentyl), Flumioxazin, Flumipropyn, Flumetsulam, Fluometuron, Fluorochloridone, Fluoroglycofen(-ethyl), Flupoxam, Flupropacil, Flurpyrsulfuron(-methyl, -sodium), Flurenol(-butyl), Fluridone, Fluroxypyr(meptyl), Flurprimidol, Flurtamone, Fluthiacet(-methyl), Fluthiamide, Fomesafen, Glufosinate(-ammonium), Glyphosate(-isopropylammonium), Halosafen, Haloxyfop-(-ethoxyethyl), Haloxyfop(-P-methyl), Hexazinone, Imazamethabenz(-methyl), Imazamethapyr, Imazamox, Imazapic, Imazapyr, Imazaquin, Imazethapyr, Imazosulfuron, Iodosulfuron(-methyl, -sodium), Ioxynil, Isopropalin, Isoproturon, Isouron, Isoxaben, Isoxachlortole, Isoxaflutole, Isoxapyrifop, Lactofen, Lenacil, Linuron, MCPA, MCPP, Mefenacet, Mesotrione, Metamitron, Metazachlor, Methabenzthiazuron, Metobenzuron, Metobromuron, (alpha-)Metolachlor, Metosulam, Metoxuron, Metribuzin, Metsulfuron(-methyl), Molinate, Monolinuron, Naproanilide, Napropamide, Neburon, Nicosulfuron, Norflurazon, Orbencarb, Oryzalin, Oxadiargyl, Oxadiazon, Oxasulfuron, Oxaziclomefone, Oxyfluorfen, Paraquat, Pelargonsäure, Pendimethalin, Pendralin, Pentoxazone, Phenmedipham, Piperophos, Pretilachlor, Primisulfuron(-methyl), Prometryn, Propachlor, Propanil, Propaquizafop, Propisochlor, Propyzamide, Prosulfocarb, Prosulfuron, Pyraflufen(-ethyl), Pyrazolate, Pyrazosulfuron(-ethyl), Pyrazoxyfen, Pyribenzoxim, Pyributicarb, Pyridate, Pyriminobac(methyl), Pyrithiobac(-sodium), Quinchlorac, Quinmerac, Quinoclamine, Quizalofop-(-P-ethyl), Quizalofop(-P-tefuryl), Rimsulfuron, Sethoxydim, Simazine, Simetryn, Sulcotrione, Sulfentrazone, Sulfometuron(-methyl), Sulfosate, Sulfosulfuron, Tebutam, Tebuthiuron, Tepraloxydim, Terbuthylazine, Terbutryn, Thenylchlor, Thiafluamide, Thiazopyr, Thidiazimin, Thifensulfuron(-methyl), Thiobencarb, Tiocarbazil, Tralkoxydim, Triallate, Triasulfuron, Tribenuron(-methyl), Triclopyr, Tridiphane, Trifluralin und Triflusulfuron.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstruktur-verbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

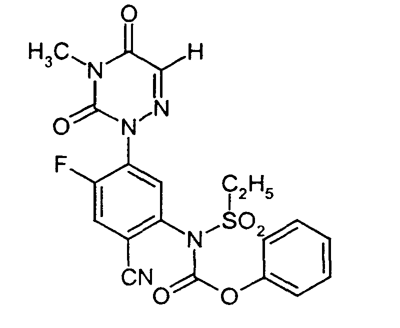

1,8 g (5 mMol) 2-(4-Cyano-2-fluor-5-ethylsulfonylamino-phenyl)-4-methyl-1,2,4-triazin-3,5(2H,4H)-dion und 1,01 g (10 mMol) Triethylamin werden in 50 ml Acetonitril mit 1,57 g (10 mMol) Chlorameisensäure-phenylester versetzt und die Reaktionsmischung wird 12 Stunden bei 25°C gerührt. Dann wird am Rotationsverdampfer eingeengt, der Rückstand mit Wasser verrührt, mit konz. Salzsäure angesäuert, ausgefallener Feststoff abgesaugt und aus Methanol umkristallisiert.

Man erhält 1,9 g (80% der Theorie) 2-[5-(N-Phenoxy-carbonyl-N-ethylsulfonylamino)-4-cyano-2-fluor-phenyl]-4-methyl-1,2,4-triazin-3,5(2H,4H)-dion vom Schmelzpunkt 181°C.
log P (pH 2): 2,76

### Beispiel 2

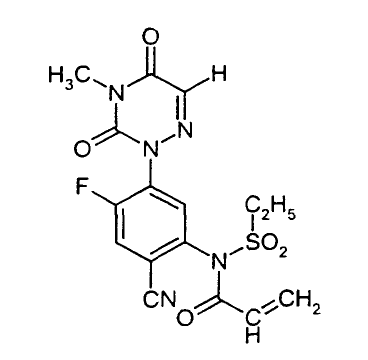

1,4 g (4 mMol) 2-(4-Cyano-2-fluor-5-ethylsulfonylamino-phenyl)-4-methyl-1,2,4-triazin-3,5(2H,4H)-dion und 0,8 g (8 mMol) Triethylamin werden in 50 ml Acetonitril mit 1,0 g (0,8 mMol) 3-Chlor-propionsäurechlorid versetzt und die Reaktionsmischung wird 12 Stunden bei 25°C gerührt. Dann wird am Rotationsverdampfer eingeengt, der Rückstand mit Wasser verrührt, mit konz. Salzsäure angesäuert, ausgefallenes Produkt abgesaugt und mit Wasser gewaschen.

Man erhält 1,4 g (86% der Theorie) 2-[5-(N-Acryloyl-N-ethylsulfonylamino)-4-cyano-2-fluor-phenyl]-4-methyl-1,2,4-triazin-3,5(2H,4H)-dion vom Schmelzpunkt 118°C.
log P (pH 2): 1,97

Analog zu den Beispielen 1 und 2 sowie entsprechend der allgemeinen Beschreibung des erfindungsgemäßen Herstellungsverfahrens können beispielsweise auch die in der nachstehenden Tabelle 1 aufgeführten Verbindungen der allgemeinen Formel (I) hergestellt werden.

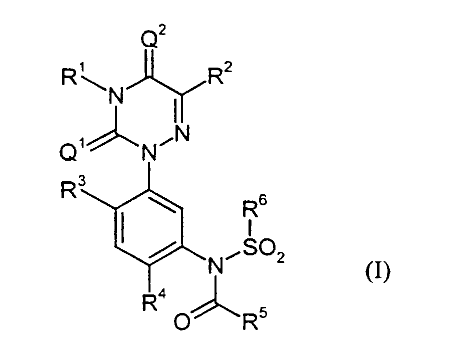

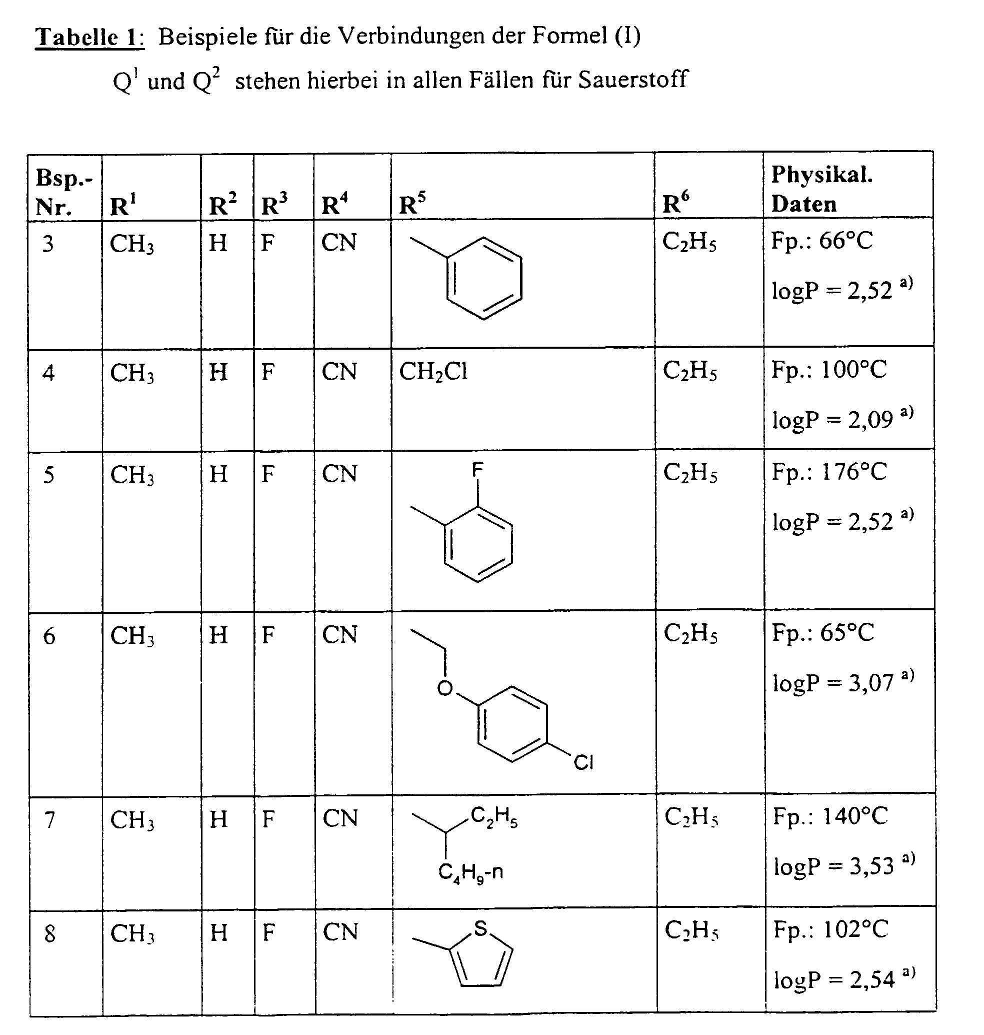

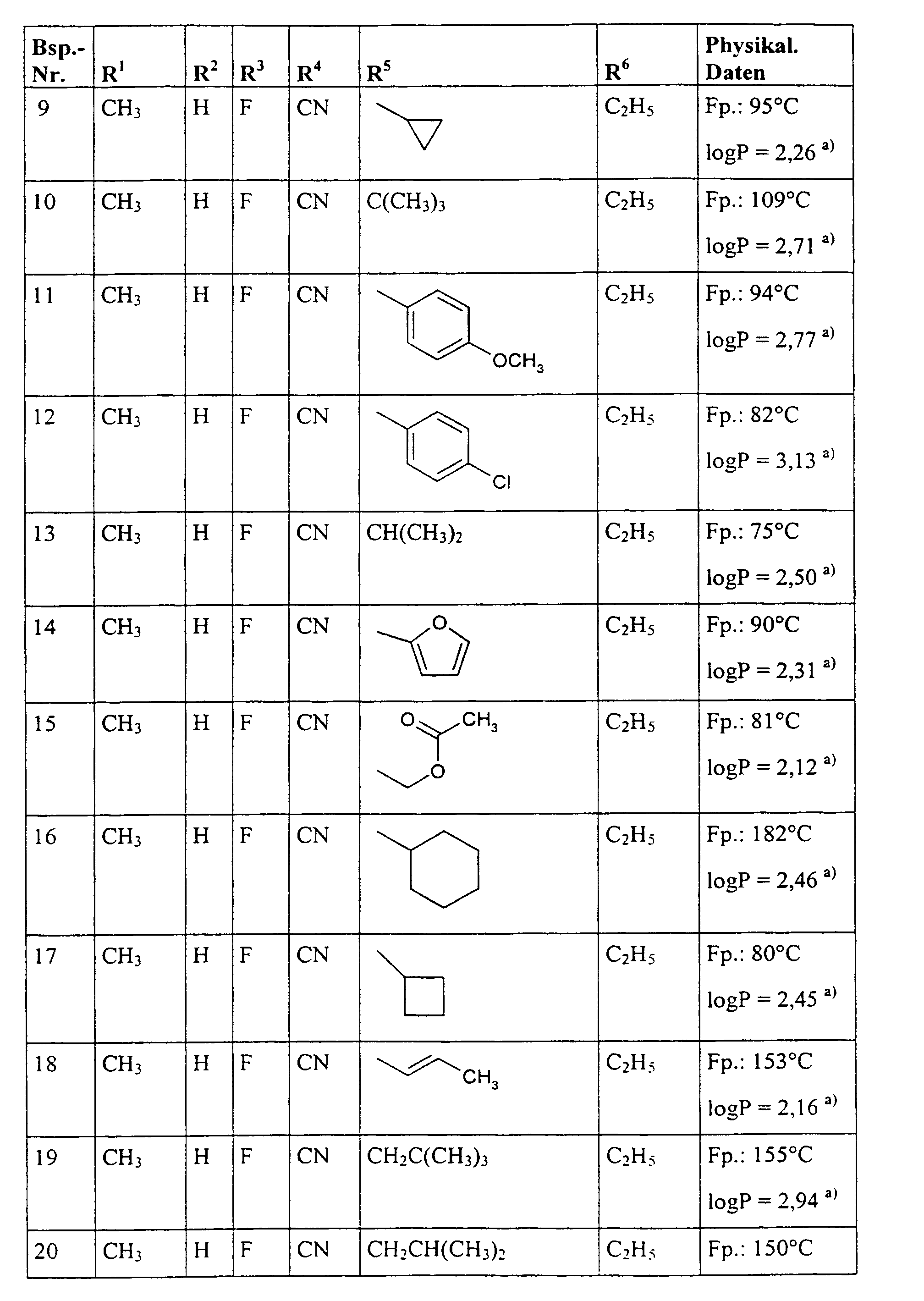

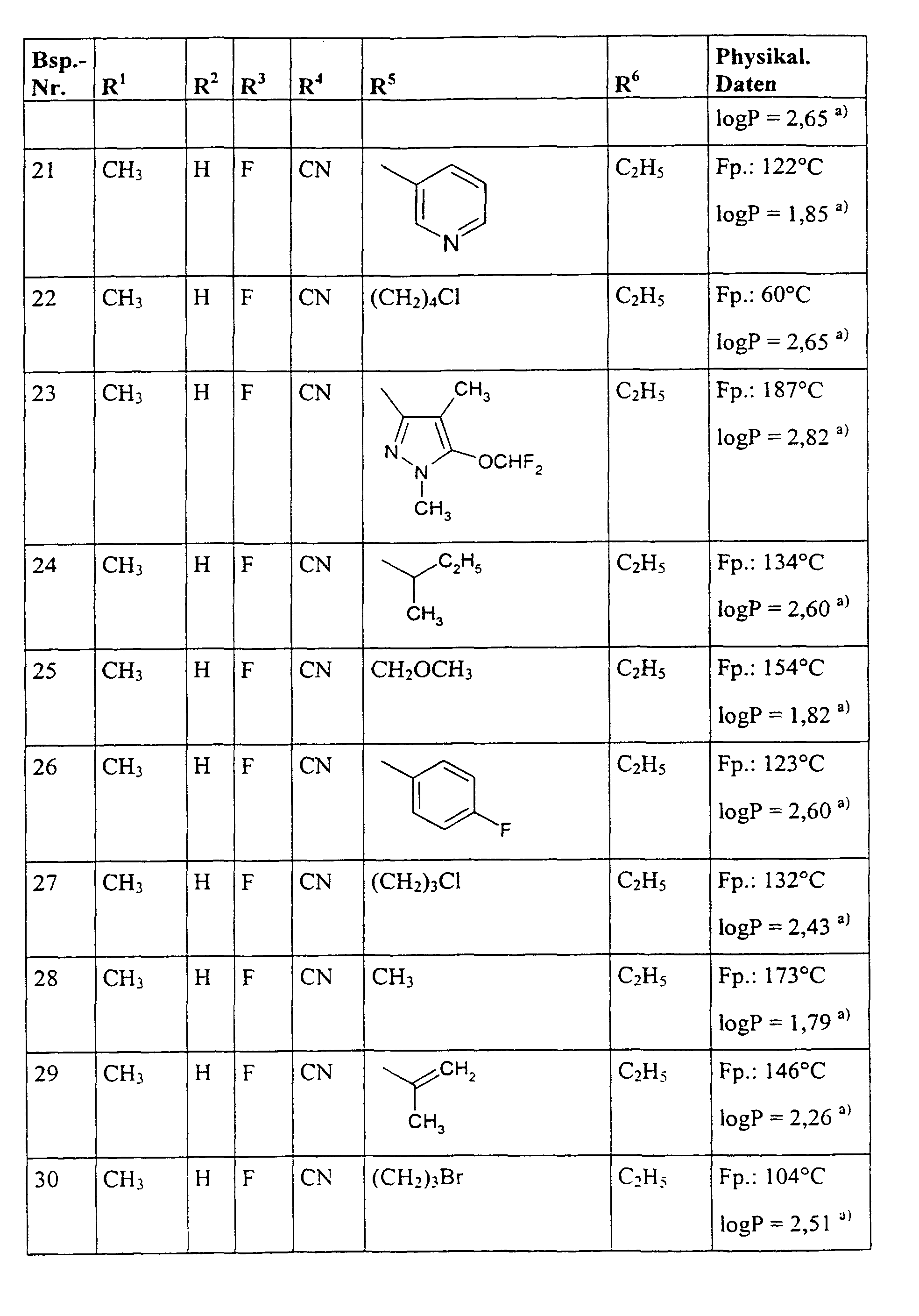

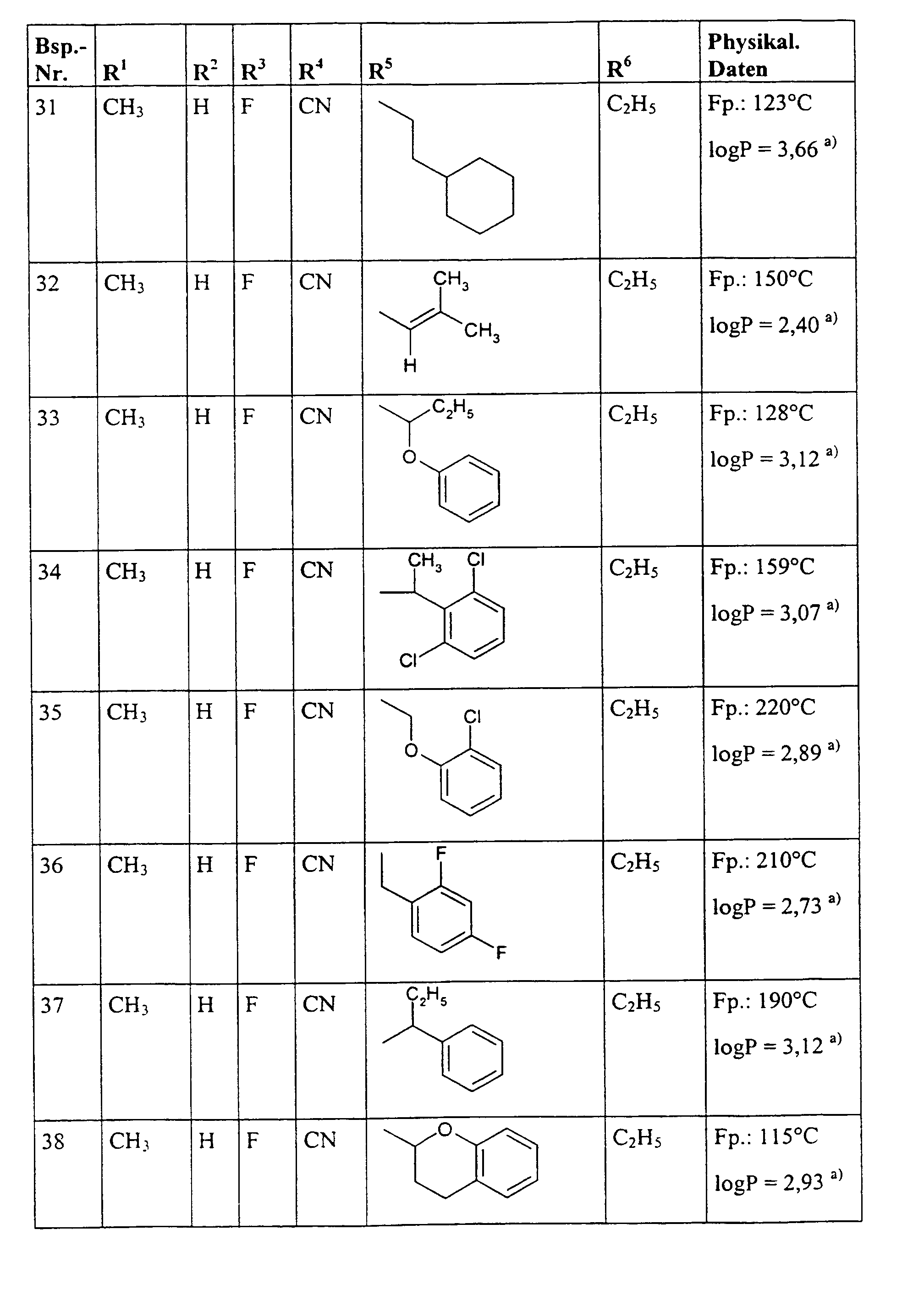

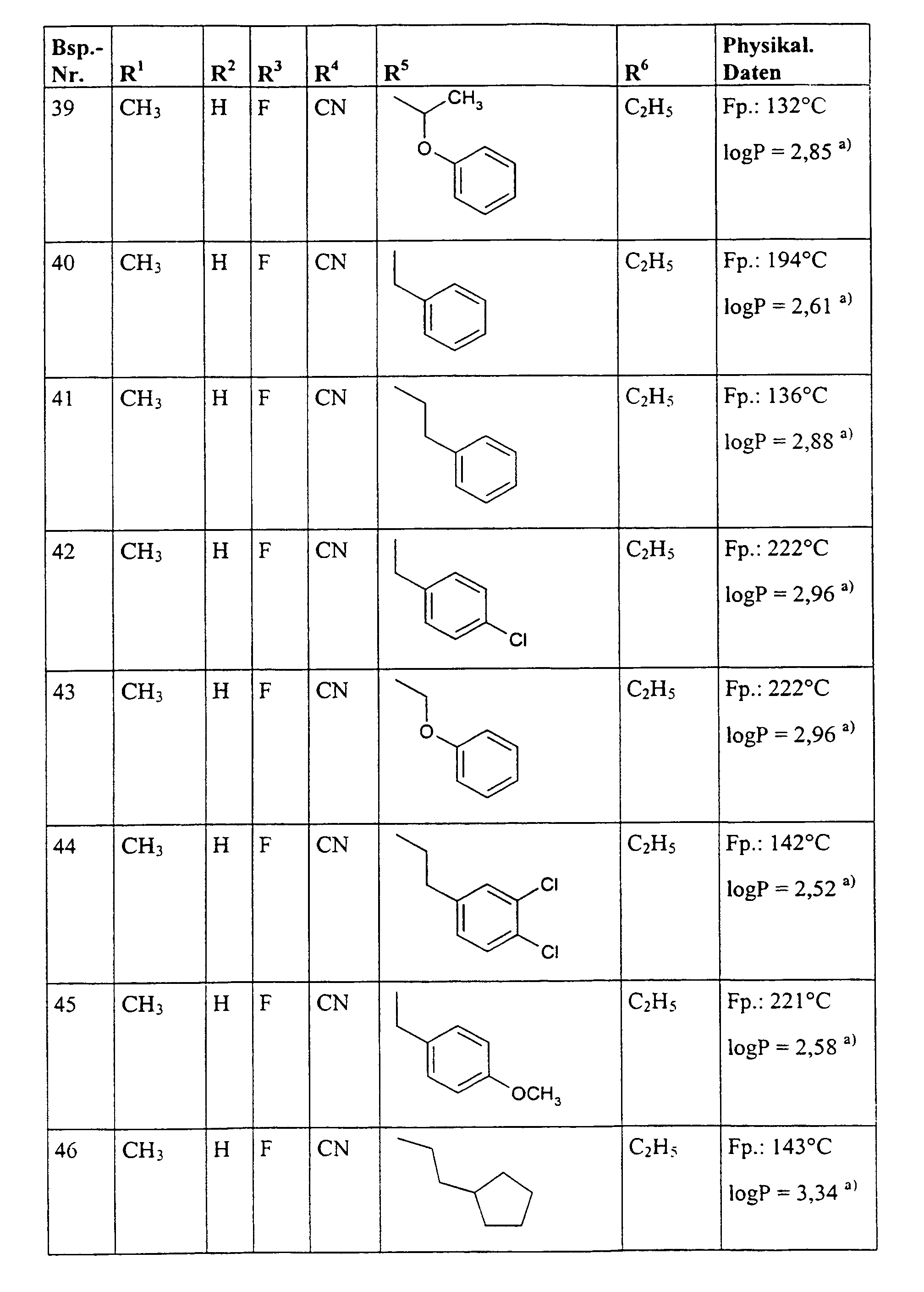

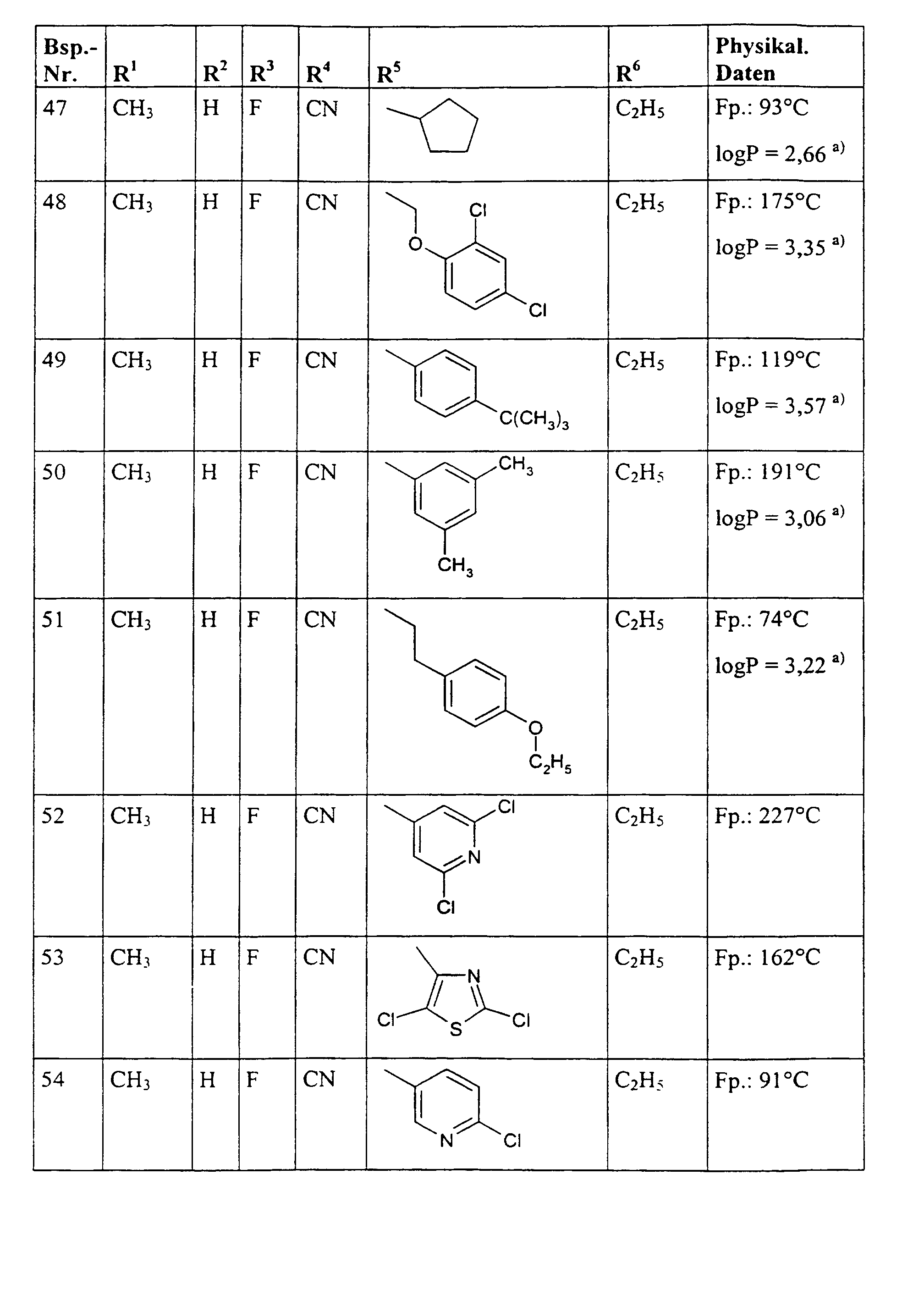

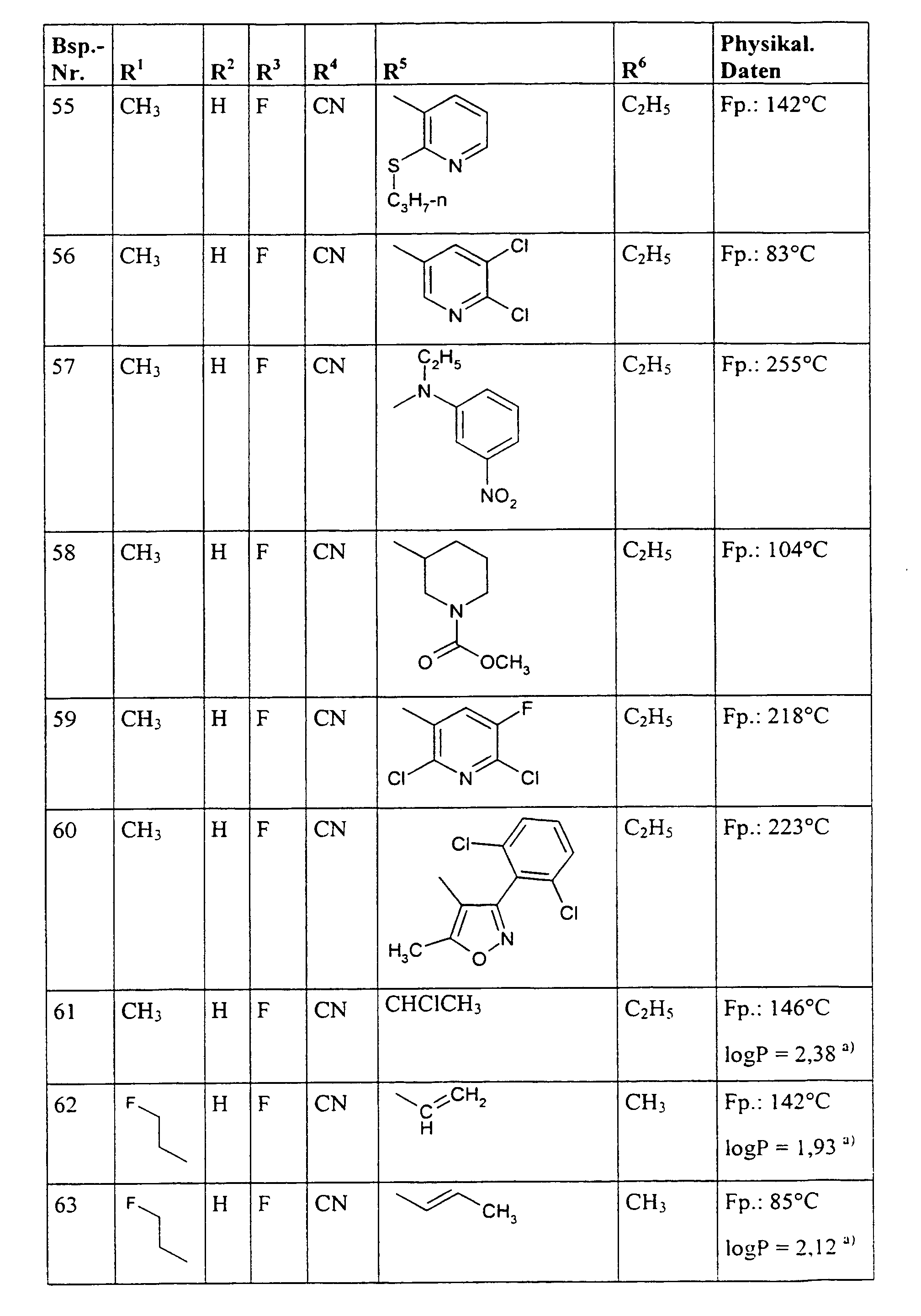

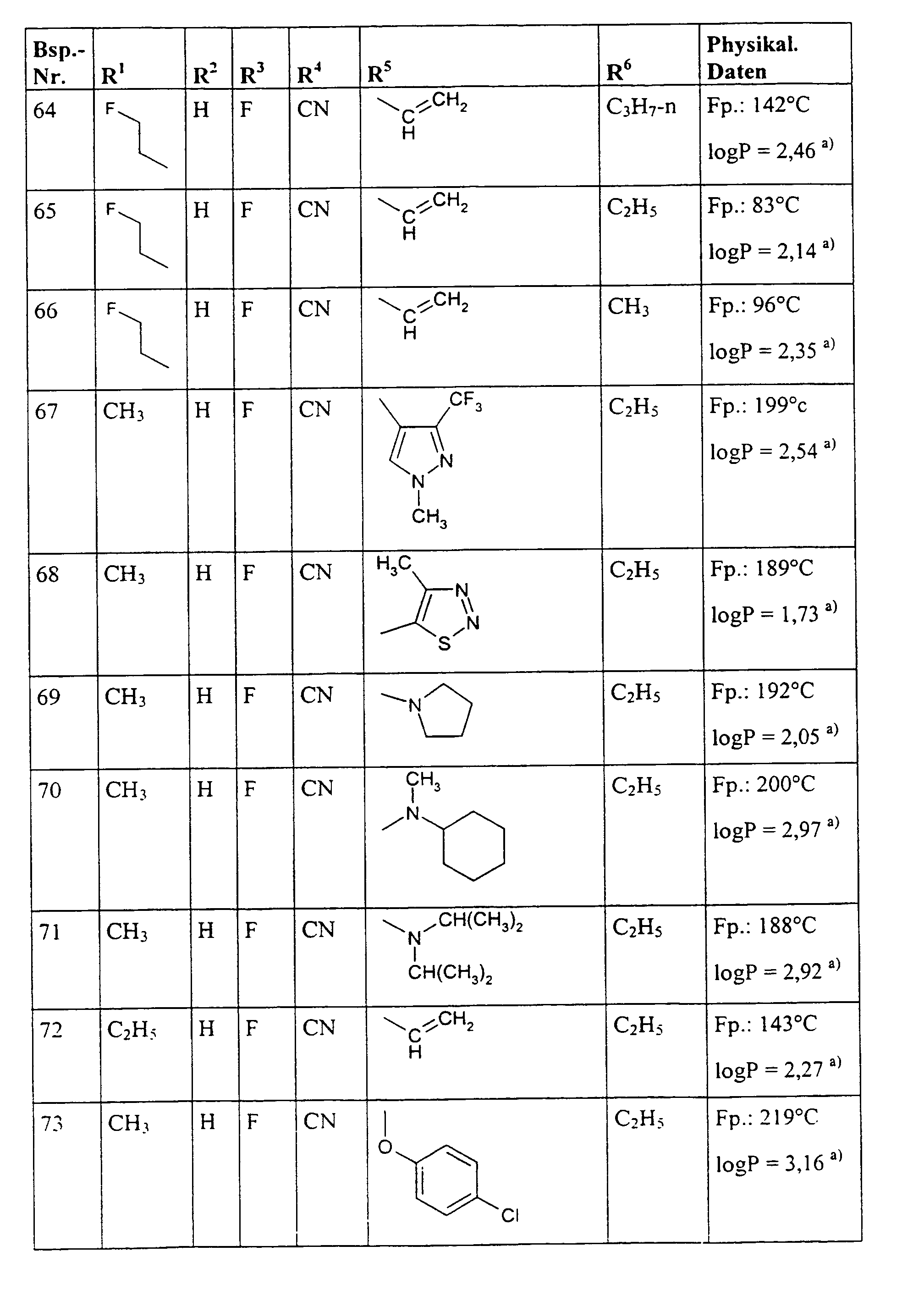

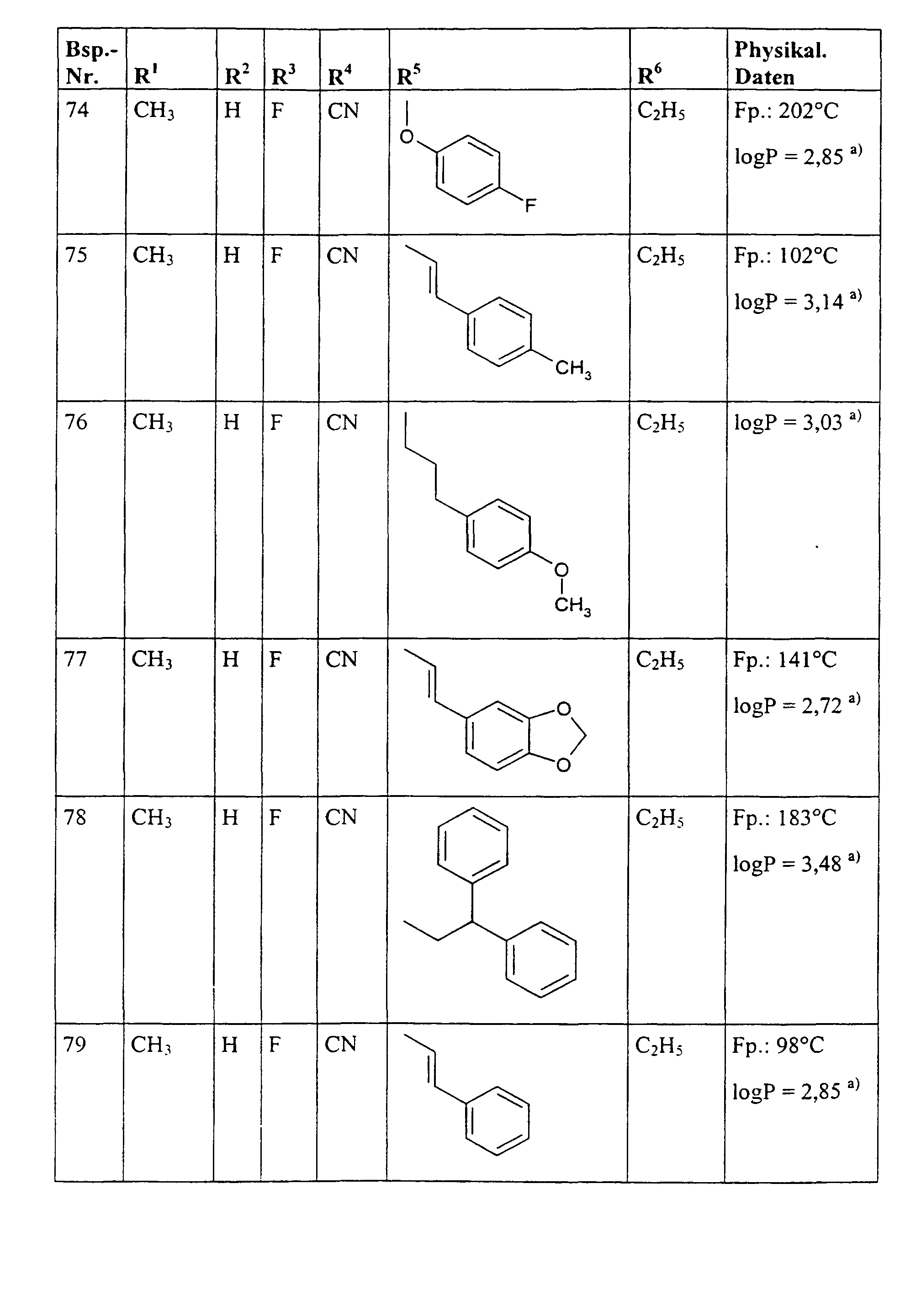

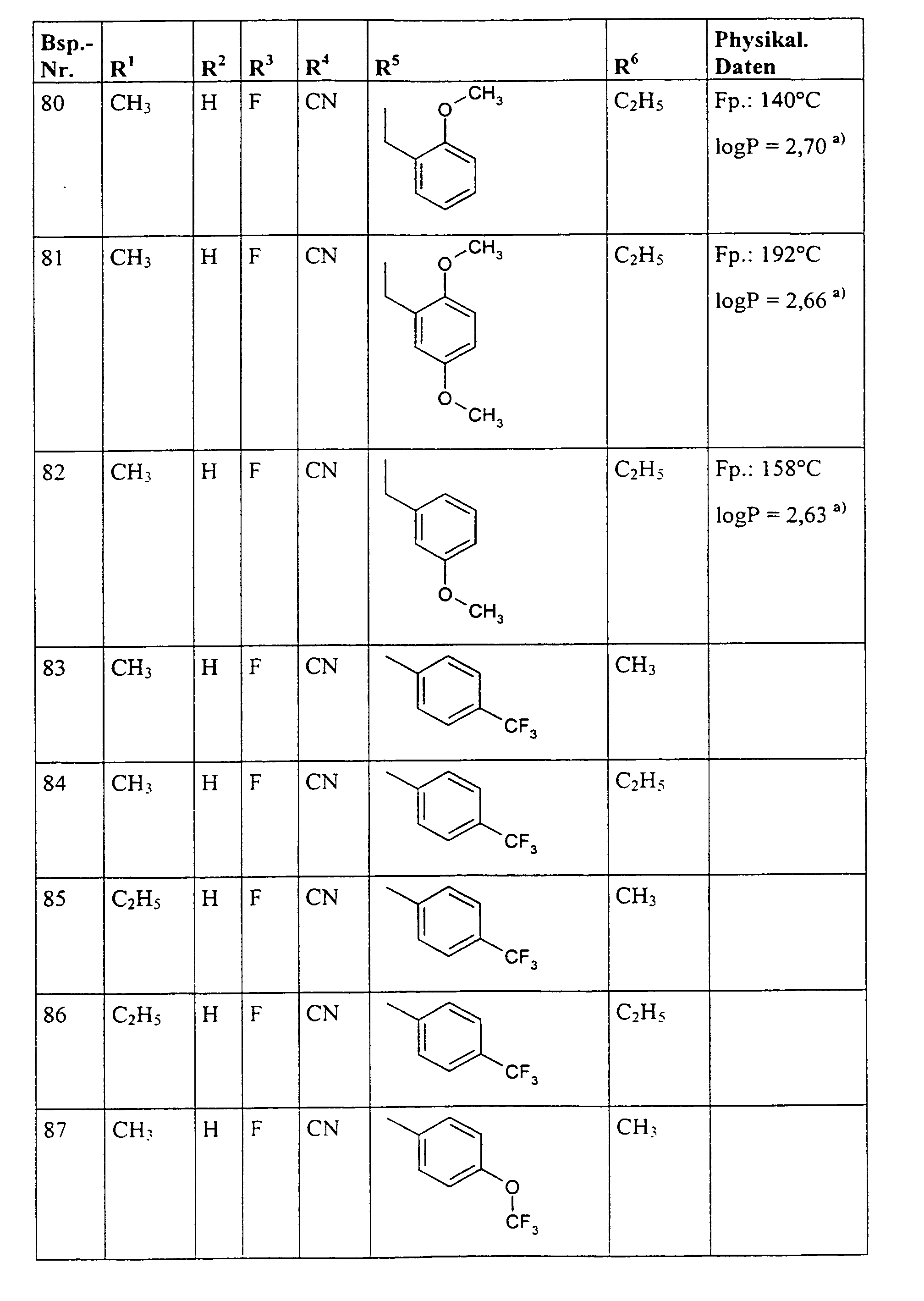

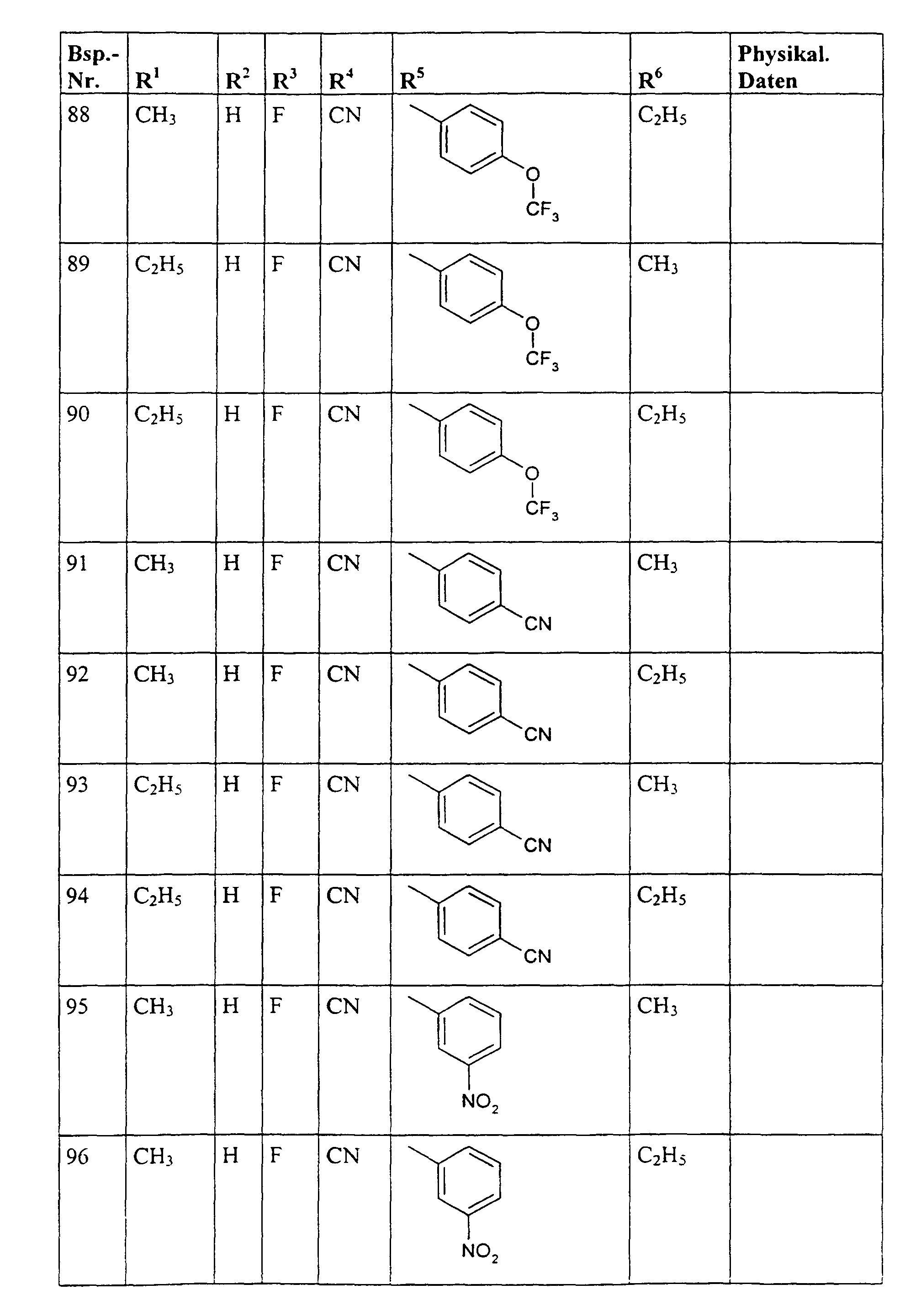

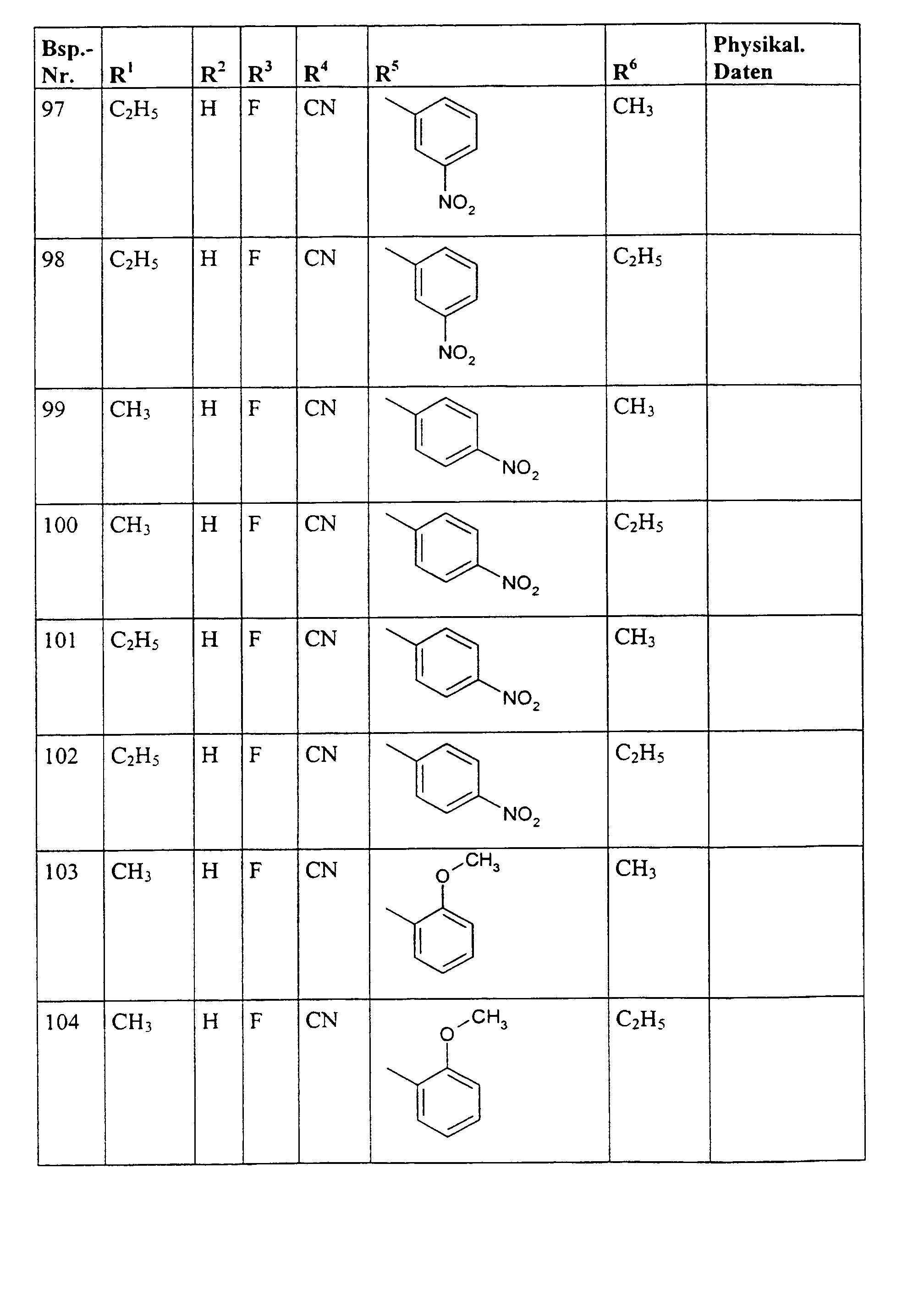

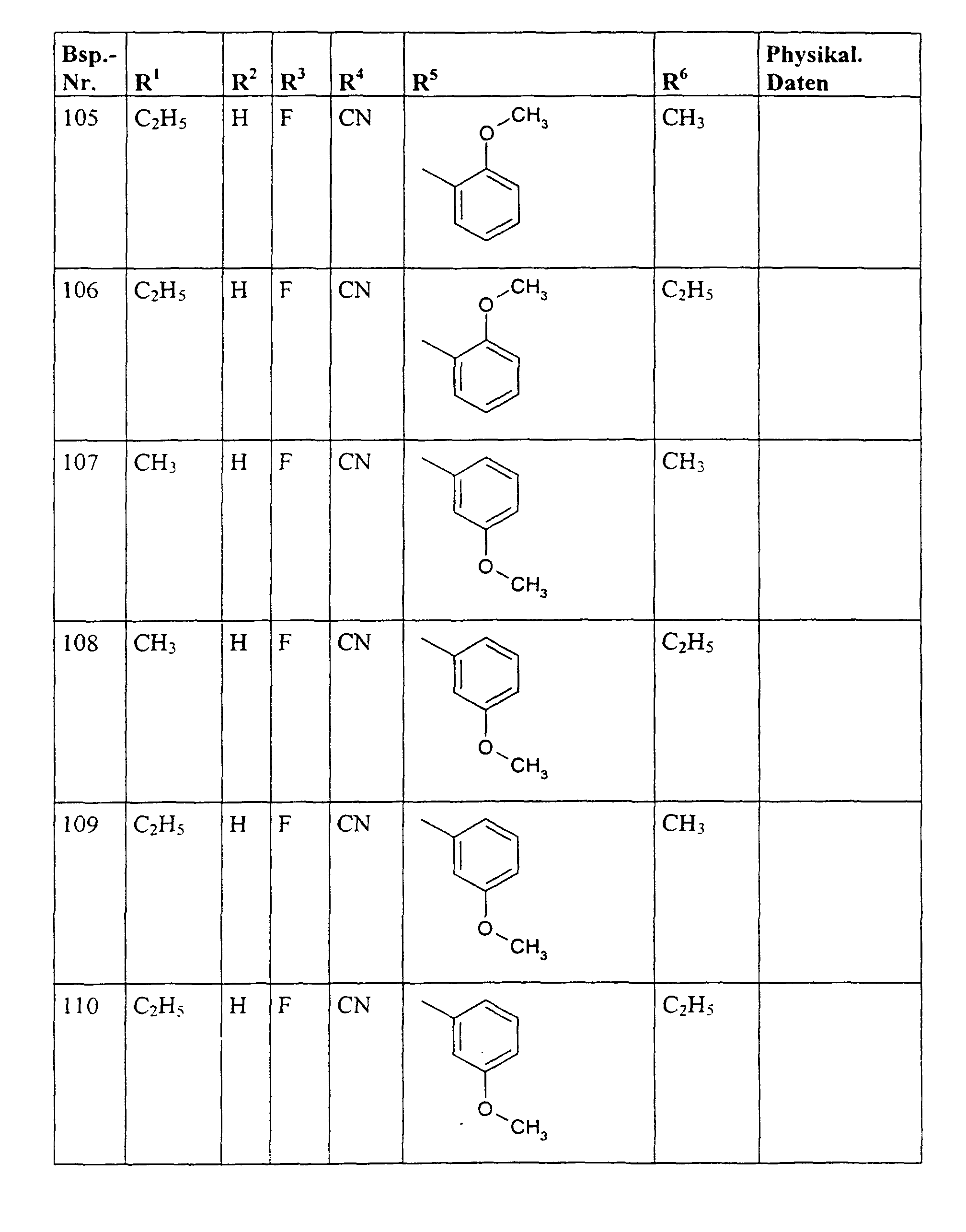

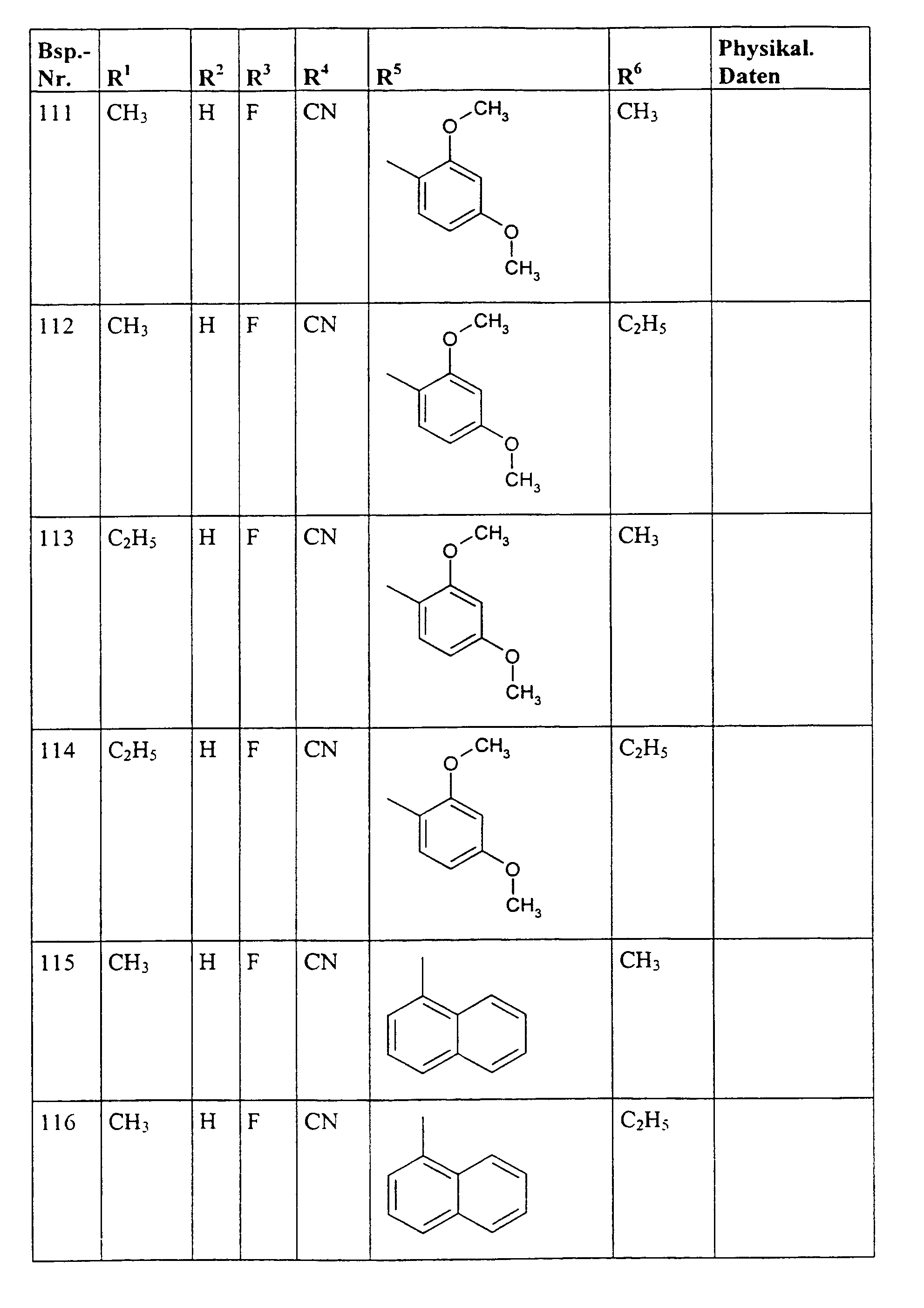

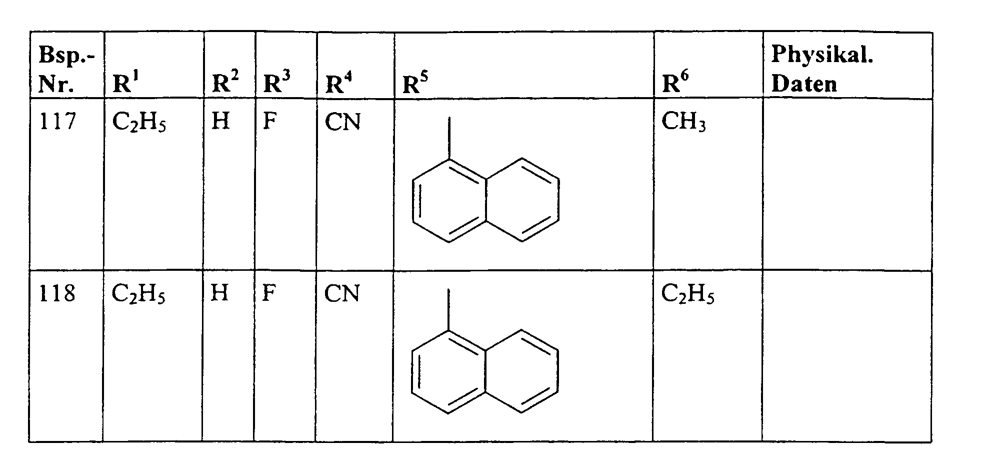

Die Bestimmung der in Tabelle 1 angegebenen logP-Werte erfolgte gemäß EEC-Directive 79/831 Annex V.A8 durch HPLC (High Performance Liquid Chromatography) an einer Phasenumkehrsäule (C 18). Temperatur: 43°C.
(a) Eluenten für die Bestimmung im sauren Bereich: 0,1% wässrige Phosphorsäure, Acetonitril; linearer Gradient von 10% Acetonitril bis 90% Acetonitril - entsprechende Messergebnisse sind in Tabelle 1 mit ^{a)} markiert.
(b) Eluenten für die Bestimmung im neutralen Bereich: 0,01-molare wässrige Phosphatpuffer-Lösung, Acetonitril; linearer Gradient von 10% Acetonitril bis 90% Acetonitril - entsprechende Messergebnisse sind in Tabelle 1 mit ^{b)} markiert.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Ausgangsstoffe der Formel (II):

### Beispiel (II-1)

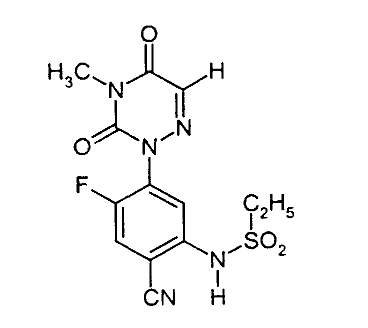

### Stufe 1

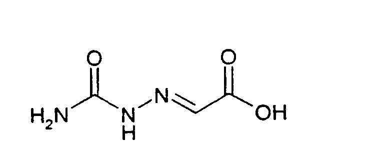

Zu 111,5 g (1 Mol) Semicarbazid-Hydrochlorid in 1 Liter Wasser lässt man zügig 162,8 g (1,1 Mol) 50%ige wässrige Glyoxalsäure zulaufen. Die Mischung wird schwach exotherm (ca. 27°C) und das Produkt fällt aus. Zur Nachreaktion wird zunächst 30 Minuten bei Raumtemperatur und anschl. 20 Minuten bei 70°C gerührt. Nach dem Abkühlen wird abgesaugt, mit Wasser und abschließend mit Aceton gewaschen.

Man erhält 128,3 g (93% der Theorie) Glyoxalsäure-semicarbazon vom Schmelzpunkt 210°C (Zers.).

### Stufe 2

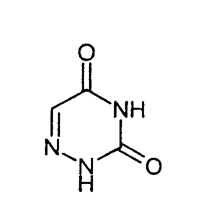

111,35 g (0,85 Mol) Glyoxalsäure-semicarbazon werden in 1000 ml 1,2-Ethandiol vorgelegt und unter Kühlung bei max. 40°C portionsweise 185 g (2,72 Mol) Natriumethanolat eingetragen. Nach beendeter exothermer Reaktion wird auf Rückflusstemperatur (ca. 110°C) erwärmt und 10 Stunden gerührt. Die abgekühlte klare Lösung wird bei 5 mbar (150°C Badtemperatur) zur Trockene eingeengt, der feste Rückstand mit ca. 1 Liter Wasser versetzt, mit konz. Salzsäure auf pH 3-2 eingestellt, nach dem Abkühlen auf 10°C, abgesaugt und mit Wasser gewaschen.

Man erhält 66,8 g (69% der Theorie) 6-Aza-uracil vom Schmelzpunkt 279°C

### Stufe 3

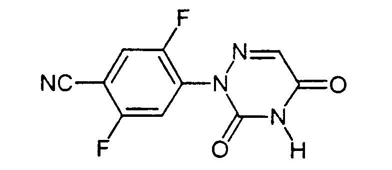

56,5 g (Mol 0,5) 6-Aza-uracil werden in 600 ml Dimethylsulfoxyd mit 69 g (0,5 Mol) Kaliumcarbonat und 83,2 g (0,53 Mol) 2,4,5-Trifluor-benzonitril versetzt und die Mischung wird 10 Stunden bei 70°C bis 80°C gerührt. Am Rotationsverdampfer wird auf ca. 1/3 des Volumens eingeengt, der Rückstand mit Wasser verrührt, mit konz. Salzsäure angesäuert, ausgefallenes Produkt abgesaugt, mit Wasser und dann mit Ethanol gewaschen.

Man erhält 116 g (92,8% der Theorie) 2-(2,5-Difluor-4-cyano-phenyl)-1,2,4-triazin-3,5(2H,4H)-dion vom Schmelzpunkt > 250°C.
log P (pH 2): 1,45

### Stufe 4

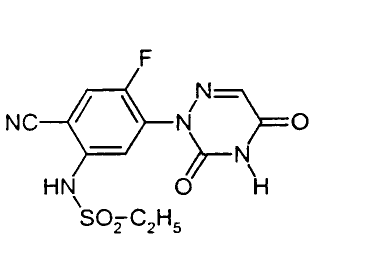

110 g (0,44 Mol) 2-(2,5-Difluor-4-cyano-phenyl)-1,2,4-triazin-3,5(2H,4H)-dion werden mit 160 g (1,16 Mol) Kaliumcarbonat und 58 g (0,52 Mol) Ethansulfonamid in 1200 ml Dimethylsulfoxid auf 125°C bis 130°C erwärmt und 14 Stunden bei dieser Temperatur gerührt. Die dunkelgrün gefärbte Suspension wird am Rotationsverdampfer zur Trockene eingeengt, der Rückstand mit Wasser verrührt, mit konz. Salzsäure langsam (Schäumen, CO₂-Entwicklung) auf pH 3-4 eingestellt, ausgefallenes Produkt abgesaugt und mit Wasser gewaschen.

Man erhält 117 g (78% der Theorie) 2-(2-Fluor-4-cyano-5-ethylsulfonylaminophenyl)-1,2,4-triazin-3,5(2H,4H)-dion vom Schmelzpunkt 121°C (Zers.).
log. P (pH 2): 1,30

### Stufe 5

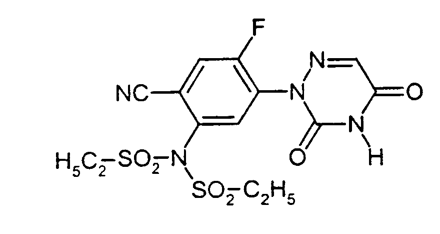

101,8 g (0,3 Mol) 2-(2-Fluor-4-cyano-5-ethylsulfonylamino-phenyl)-1,2,4-triazin-3,5(2H,4H)-dion werden mit 47,0 g (0,34 Mol) Kaliumcarbonat in 1100 ml Acetonitril vorgelegt und 41,0 g (0.32 Mol) Ethansulfonsäurechlorid zügig dazu gegeben. Anschließend wird die Mischung 12 Stunden bei Raumtemperatur gerührt, im Wasserstrahlvakuum eingeengt, der Rückstand mit Wasser verrührt, mit konz. Salzsäure auf pH 2-3 eingestellt, abgesaugt, mit Wasser und Isopropanol gewaschen und getrocknet.

Man erhält 106,2 g (76% der Theorie) 2-(2-Fluor-4-cyano-5-ethylsulfonylaminophenyl)-1,2,4-triazin-3,5(2H,4H)-dion vom Schmelzpunkt >250°C.
log P (pH 2): 2,07

### Stufe 6

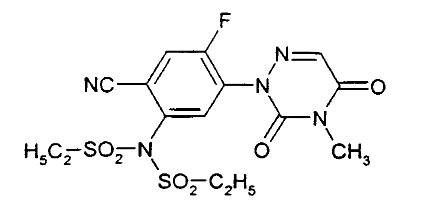

38,8 g (0,09 Mol) 2-(2-Fluor-4-cyano-5-ethylsulfonylamino-phenyl)-1,2,4-triazin-3,5(2H,4H)-dion werden mit 13,8 g (0,1 Mol) Kaliumcarbonat in 700 ml Acetonitril mit 14,2 g (0,1 Mol) Methyliodid 12 Stunden bei Raumtemperatur (ca. 20°C) gerührt. Die Mischung wird eingeengt, der Rückstand mit Wasser verrührt, mit konz. Salzsäure angesäuert, das ausgefallene Produkt abgesaugt und mit Wasser gewaschen.

Man erhält 38 g (94% der Theorie) 2-[2-Fluor-4-cyano-5-(bis-ethylsulfonyl)-aminophenyl]-4-methyl-1,2,4-triazin-3,5(2H,4H)-dion vom Schmelzpunkt 164°C.
log P (pH 2): 2,24

### Stufe 7

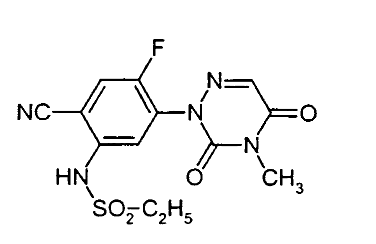

45,8 g (0,103 Mol) 2-[2-Fluor-4-cyano-5-(bis-ethylsulfonyl)-amino-phenyl]-4-methyl-1,2,4-triazin-3,5(2H,4H)-dion werden in 500 ml Aceton und 200 ml Wasser mit 18 g (0,22 Mol) Natriumhydrogencarbonat 12 Stunden bei Rückflusstemperatur gerührt und anschließend im Wasserstrahlvakuum eingeengt. Der Rückstand wird in Wasser verrührt, mit konz. Salzsäure angesäuert, ausgefallenes Produkt abgesaugt, mit Wasser gewaschen, anschließend mit Ethanol verrührt, abgesaugt, gewaschen und getrocknet.

Man erhält 32,3 g (89% der Theorie) 2-(2-Fluor-4-cyano-5-ethylsulfonylaminophenyl)-4-methyl-1,2,4-triazin-3,5(2H,4H)-dion vom Schmelzpunkt 233°C.
log P (pH 2): 1,66

### Anwendungsbeispiele:

### Beispiel A

| Pre-emergence-test | |
|---|---|
| Lösungsmittel | 5 Gewichtsteile Aceton |
| Emulgator | 1 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät. Nach ca. 24 Stunden wird der Boden so mit der Wirkstoffzubereitung besprüht, dass die jeweils gewünschte Wirkstoffmenge pro Flächeneinheit ausgebracht wird. Die Konzentration der Spritzbrühe wird so gewählt, dass in 1000 Liter Wasser pro Hektar die jeweils gewünschte Wirkstoffmenge ausgebracht wird.

Nach drei Wochen wird der Schädigungsgrad der Pflanzen bonitiert in % Schädigung im Vergleich zur Entwicklung der unbehandelten Kontrolle.

Es bedeuten:
0% = keine Wirkung (wie unbehandelte Kontrolle)
100% = totale Vernichtung

In diesem Test zeigen beispielsweise die Verbindungen gemäß Herstellungsbeispiel 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 35, 36, 37, 39, 40, 41, 42, 43, 44, 45, 46, 47, 48, 49, 50, 51 und 61 bei weitgehend guter Verträglichkeit gegenüber Kulturpflanzen, wie z.B. Gerste, Weizen, Raps, Soja und Zuckerrüben, sehr starke Wirkung gegen Unkräuter.

### Beispiel B

| Post-emergence-Test | |
|---|---|
| Lösungsmittel | 5 Gewichtsteile Aceton |
| Emulgator | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, dass die jeweils gewünschten Wirkstoffmengen pro Flächeneinheit ausgebracht werden. Die Konzentration der Spritzbrühe wird so gewählt, dass in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.

Es bedeuten:
0 % = keine Wirkung (wie unbehandelte Kontrolle)
100 % = totale Vernichtung

In diesem Test zeigen beispielsweise die Verbindungen gemäß Herstellungsbeispiel 2, 3, 4, 5, 6, 7, 8, 9, 10, 12, 13, 14, 16, 17, 18, 19, 20, 21, 22, 23, 24, 25, 26, 27, 28, 29, 30, 31, 32, 33, 35, 36, 37, 39, 40, 41, 42, 43, 44, 45, 46, 47, 48, 49, 50, 51 und 61 bei weitgehend guter Verträglichkeit gegenüber Kulturpflanzen, wie z.B. Gerste und Weizen, sehr starke Wirkung gegen Unkräuter.

## Patentansprüche

1. Verbindungen der allgemeinen Formel (I) 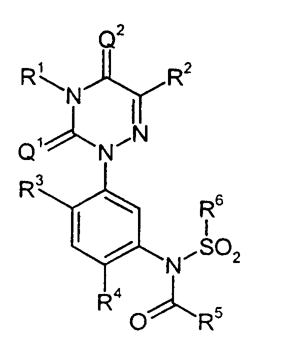 in welcher
Q¹ für Sauerstoff oder Schwefel steht,
Q² für Sauerstoff oder Schwefel steht,
R¹ für Wasserstoff, Cyano, Amino, für jeweils gegebenenfalls durch Cyano, Halogen oder C₁-C₄-Alkoxy substituiertes Alkyl, Alkoxy, Alkylamino, Dialkylamino, Alkylcarbonyl oder Alkoxycarbonyl mit jeweils 1 bis 6 Kohlenstoffatomen in den Alkylgruppen, für jeweils gegebenenfalls durch Halogen substituiertes Alkenyl, Alkenylcarbonyl, Alkenyloxycarbonyl, Alkinyl, Alkinylcarbonyl oder Alkinyloxycarbonyl mit jeweils 2 bis 6 Kohlenstoffatomen in den Alkenyl- bzw. Alkinyl-gruppen, oder für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 4 Kohlenstoffatomen im Alkylteil steht,
R² für Wasserstoff, Halogen, Nitro, Carboxy, Cyano, Thiocarbamoyl, Amino, für jeweils gegebenenfalls durch Cyano, Halogen oder C₁-C₄-Alkoxy substituiertes Alkyl, Alkoxy, Alkylthio, Alkylamino oder Di-alkylamino mit jeweils 1 bis 6 Kohlenstoffatomen in den Alkylgruppen, für jeweils gegebenenfalls durch Halogen substituiertes Alkenyl, Alkenyloxy, Alkenylthio, Alkinyl, Alkinyloxy oder Alkinylthio mit jeweils 2 bis 6 Kohlenstoffatomen in den Alkenyl- oder Alkinyl-gruppen, oder für jeweils gegebenenfalls durch Cyano, Halogen oder C₁-C₄-Alkyl substituiertes Cycloalkyl oder Cycloalkylalkyl mit jeweils 3 bis 6 Kohlenstoffatomen in den Cycloalkylgruppen und gegebenenfalls 1 bis 6 Kohlenstoffatomen im Alkylteil steht,
R³ für Wasserstoff, Cyano oder Halogen steht,
R⁴ für Cyano oder Thiocarbamoyl steht,
R⁵ für Wasserstoff, für Alkoxycarbonyl mit 1 bis 6 Kohlenstoffatomen in der Alkoxygruppe, oder für einen der Reste -R⁷, -O-R⁷, -SR⁷, -NH-R⁷ oder -NR⁷R⁸ steht,
R⁶ für Amino, Hydroxy oder für einen der Reste -R⁷ oder -NR⁷R⁸ steht,
R⁷ für gegebenenfalls einfach oder mehrfach, gleich oder verschieden substituiertes, geradkettiges oder verzweigtes Alkyl mit 1 bis 10 Kohlenstoffatomen steht, wobei als Substituenten in Betracht kommen:
Cyano, Carboxy, Carbamoyl, Thiocarbamoyl, Halogen, jeweils geradkettiges oder verzweigtes Alkoxy, Alkoxyalkoxy, Alkylcarbonyloxy, Alkylthio, Alkylsulfinyl, Alkylsulfonyl, Alkoxycarbonyl, N-Alkylaminocarbonyl, N,N-Dialkyl-aminocarbonyl, Trialkylsilyl oder Alkylsulfonylaminocarbonyl mit jeweils 1 bis 6 Kohlenstoffatomen in den einzelnen Alkylteilen, oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht, oder
R⁷ für jeweils gegebenenfalls einfach oder mehrfach, gleich oder verschieden durch Halogen substituiertes Alkenyl oder Alkinyl mit jeweils 2 bis 8 Kohlenstoffatomen steht, oder
R⁷ für jeweils gegebenenfalls einfach oder mehrfach, gleich oder verschieden durch Cyano, Halogen und/oder C₁-C₄-Alkyl substituiertes Cycloalkyl oder Cycloalkylalkyl mit jeweils 3 bis 7 Kohlenstoffatomen in den Cycloalkylgruppen und gegebenenfalls 1 bis 4 Kohlenstoffatomen im Alkylteil steht, oder
R⁷ für jeweils gegebenenfalls im Arylteil einfach oder mehrfach, gleich oder verschieden substituiertes Aryl, Bis-Aryl-alkyl, Arylalkenyl, Arylalkyl, Aryloxyalkyl oder Arylalkoxyalkyl mit jeweils 6 bis 10 Kohlenstoffatomen im Arylteil und gegebenenfalls bis zu 4 Kohlenstoffatomen im geradkettigen oder verzweigten Alkylteil bzw. Alkenylteil steht, oder für einen gegebenenfalls einfach oder mehrfach, gleich oder verschieden substituierten und/oder benzannellierten, gesättigten oder ungesättigten, fünf- bis siebengliedrigen Heterocyclylrest mit 1 bis 3 gleichen oder verschiedenen Heteroatomen - insbesondere Stickstoff, Sauerstoff und/oder Schwefel - steht, wobei als Aryl- bzw. Heterocyclylsubstituenten in Betracht kommen:
Halogen, Cyano, Nitro, Amino, Methylendioxy, N-(C₁-C₄-Alkylcarbonyl)-amino, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 3 gleichen oder verschiedenen Halogenatomen, jeweils geradkettiges oder verzweigtes Alkoxycarbonyl oder Alkoximinoalkyl mit jeweils 1 bis 6 Kohlenstoffatomen in den einzelnen Alkylteilen sowie gegebenenfalls einfach oder mehrfach, gleich oder verschieden durch Halogen und/oder geradkettiges oder verzweigtes Alkyl oder Alkoxy mit jeweils 1 bis 6 Kohlenstoffatomen und/oder geradkettiges oder verzweigtes Halogenalkyl oder Halogenalkoxy mit jeweils 1 bis 6 Kohlenstoffatomen und 1 bis 3 gleichen oder verschiedenen Halogenatomen substituiertes Phenyl, und
R⁸ für Wasserstoff oder für gegebenenfalls einfach oder mehrfach, gleich oder verschieden substituiertes, geradkettiges oder verzweigtes Alkyl mit 1 bis 8 Kohlenstoffatomen steht, wobei als Substituenten in Betracht kommen:
Cyano, Carboxy, Carbamoyl, Thiocarbamoyl, Halogen, jeweils geradkettiges oder verzweigtes Alkoxy, Alkoxyalkoxy, Alkylthio, Alkylsulfinyl, Alkylsulfonyl, Alkoxycarbonyl, Alkylaminocarbonyl, Dialkylaminocarbonyl, Trialkylsilyl oder Alkylsulfonylaminocarbonyl mit jeweils 1 bis 8 Kohlenstoffatomen in den einzelnen Alkylteilen oder Heterocyclyl, wobei als Heterocyclylrest ein fünf- bis siebengliedriger, gegebenenfalls benzannellierter, gesättigter oder ungesättigter Heterocyclus mit 1 bis 3 gleichen oder verschiedenen Heteroatomen - insbesondere Stickstoff, Sauerstoff und/oder Schwefel - steht, oder
R⁸ für jeweils gegebenenfalls einfach oder mehrfach, gleich oder verschieden durch Halogen substituiertes Alkenyl oder Alkinyl mit jeweils 2 bis 8 Kohlenstoffatomen steht, oder
R⁸ für gegebenenfalls einfach oder mehrfach, gleich oder verschieden durch Cyano, Halogen und/oder geradkettiges oder verzweigtes Alkyl mit 1 bis 4 Kohlenstoffatomen substituiertes Cycloalkyl mit 3 bis 7 Kohlenstoffatomen steht, oder
R⁸ zusammen mit R⁷ für gegebenenfalls durch O (Sauerstoff), S (Schwefel), NH oder N(C₁-C₄-Alkyl) unterbrochenes Alkandiyl (Alkylen) mit 2 bis 6 Kohlenstoffatomen steht.

2. Verbindungen gemäß Anspruch 1, **dadurch gekennzeichnet, dass**
R¹ für Wasserstoff, Cyano, Amino, für jeweils gegebenenfalls durch Cyano, Fluor, Chlor, Methoxy oder Ethoxy substituiertes Methyl, Ethyl, n- oder i-Propyl, n-, i- s- oder t-Butyl, Methoxy, Ethoxy, n- oder i-Propoxy, n-, i-, s- oder t-Butoxy, Methylamino, Ethylamino, n- oder i-Propylamino, n-, i-, s- oder t-Butylamino, Dimethylamino, Di-ethylamino, Acetyl, Propionyl, n- oder i-Butyroyl, Methoxycarbonyl, Ethoxycarbonyl, n- oder i-Propoxycarbonyl, für jeweils gegebenenfalls durch Fluor, Chlor oder Brom substituiertes Propenyl, Butenyl, Propenylcarbonyl, Butenylcarbonyl, Propenyloxycarbonyl, Butenyloxycarbonyl, für Propinyl, Butinyl, Propinylcarbonyl, Butinylcarbonyl, Propinyloxycarbonyl oder Butinyloxycarbonyl, oder für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R² für Wasserstoff, Nitro, Carboxy, Cyano, Thiocarbamoyl, Amino, Fluor, Chlor, Brom, für jeweils gegebenenfalls durch Cyano, Fluor, Chlor, Methoxy oder Ethoxy substituiertes Methyl, Ethyl, n- oder i-Propyl, n-, i- s- oder t-Butyl, Methoxy, Ethoxy, n- oder i-Propoxy, n-, i-, s- oder t-Butoxy, Methylthio, Ethylthio, n- oder i-Propylthio, n-, i-, s- oder t-Butylthio, Methylamino, Ethylamino, n- oder i-Propylamino, n-, i-, s- oder t-Butylamino, Dimethylamino oder Diethylamino, für jeweils gegebenenfalls durch Fluor, Chlor oder Brom substituiertes Propenyl, Propenyloxy, Propenylthio, Butenyl, Butenyloxy oder Butenylthio, für Propinyl, Propinyloxy, Propinylthio, Butinyl, Butinyloxy oder Butinylthio, oder für jeweils gegebenenfalls durch Cyano, Fluor, Chlor, Brom, Methyl oder Ethyl substituiertes Cyclopropyl, Cyclobutyl, Cyclopentyl, Cyclohexyl, Cyclopropylmethyl, Cyclobutylmethyl, Cyclopentylmethyl oder Cyclohexylmethyl steht,
R³ für Wasserstoff, Fluor oder Chlor steht,
R⁵ für Wasserstoff, Methoxycarbonyl, Ethoxycarbonyl, n- oder i-Propoxycarbonyl, oder für einen der Reste -R⁷, -O-R⁷, -S-R⁷, -NH-R⁷ oder -NR⁷R⁸ steht,
R⁷ für jeweils gegebenenfalls einfach oder zweifach substituiertes Methyl, Ethyl, n- oder i-Propyl, n-, i-, s- oder t-Butyl, n-, i-, s-, t- oder neo-Pentyl, n-, i- oder s-Hexyl steht, wobei als Substituenten jeweils vorzugsweise in Betracht kommen:
Cyano, Carboxy, Carbamoyl, Fluor, Chlor, Brom, Methoxy, Ethoxy, n- oder i-Propoxy, Methoxymethoxy, Ethoxymethoxy, Methoxyethoxy, Ethoxyethoxy, Acetylmethoxy, Methylthio, Ethylthio, n- oder i-Propylthio, Methylsulfinyl, Ethylsulfinyl, Methylsulfonyl, Ethylsulfonyl, Methoxycarbonyl, Ethoxycarbonyl, Methylaminocarbonyl, Ethyl-aminocarbonyl, Dimethylaminocarbonyl, Trimethylsilyl, Methylsulfonylaminocarbonyl oder Ethylsulfonylaminocarbonyl, oder
R⁷ für jeweils gegebenenfalls einfach oder zweifach durch Fluor und/oder Chlor substituiertes Ethenyl, Propenyl, Butenyl, Ethinyl, Propinyl oder Butinyl steht, oder
R⁷ für jeweils gegebenenfalls einfach oder zweifach, gleich oder verschieden durch Fluor, Chlor, Methyl und/oder Ethyl substituiertes Cyclopropyl, Cyclobutyl, Cyclopentyl, Cyclohexyl, Cyclopropylmethyl, Cyclopropylethyl, Cyclopentylmethyl, Cyclopentylethyl, Cyclohexylmethyl oder Cyclohexylethyl steht, oder
R⁷ für jeweils gegebenenfalls einfach, zweifach oder dreifach, gleich oder verschieden substituiertes Phenyl, Benzyl, Phenylethyl, Phenylpropyl, Phenoxymethyl, 2,2-Bis-phenyl-ethyl, Phenylethenyl, Phenoxyethyl, Phenoxypropyl, Furyl, Furylmethyl, Thienyl, Oxazolyl, Isoxazolyl, Thiazolyl, Pyrazolyl, Oxadiazolyl, Thiadiazolyl, Pyridinyl, Pyridinylmethyl, Pyrimidinyl, Pyrrolidinyl, Piperidinyl, Morpholinyl oder Chromanyl steht, wobei als Substituenten jeweils vorzugsweise in Betracht kommen:
Fluor, Chlor, Brom, Cyano, Nitro, Amino, Methylendioxy, N-Acetylamino, Methyl, Ethyl, n- oder i-Propyl, n-, i-, s- oder t-Butyl, Methoxy, Ethoxy, n- oder i-Propoxy, n-, i-, s- oder t-Butoxy, Methylthio, Ethylthio, Methylsulfinyl, Methylsulfonyl, Trifluormethyl, Difluormethyl, Trifluormethoxy, Difluormethoxy, Trifluormethylthio, Trifluormethylsulfinyl, Trifluormethylsulfonyl, Methoxycarbonyl, Ethoxycarbonyl, Methoximinomethyl, Methoximinoethyl, Ethoximinomethyl, Ethoximinoethyl, und
R⁸ für Wasserstoff oder für jeweils gegebenenfalls einfach substituiertes Methyl, Ethyl, n- oder i-Propyl, n- oder i-Butyl steht, wobei als Substituenten vorzugsweise in Betracht kommen:
Cyano, Carboxyl, Carbamoyl, Fluor, Chlor, Methoxy, Ethoxy, n- oder i-Propoxy, Methoxymethoxy, Ethoxymethoxy, Methoxyethoxy, Ethoxyethoxy, Methylthio, Ethylthio, n- oder i-Propylthio, Methylsulfinyl, Ethylsulfinyl, Methylsulfonyl, Ethylsulfonyl, Methoxycarbonyl, Ethoxycarbonyl, Methylaminocarbonyl, Ethylaminocarbonyl, Dimethylaminocarbonyl, oder
R⁸ für jeweils gegebenenfalls einfach oder zweifach durch Fluor und/oder Chlor - substituiertes Ethenyl, Propenyl, Butenyl, Ethinyl, Propinyl oder Butinyl steht, oder
R⁸ für gegebenenfalls einfach oder zweifach, gleich oder verschieden durch Fluor, Chlor, Methyl und/oder Ethyl substituiertes Cyclopropyl, Cyclopentyl oder Cyclohexyl steht, oder
R⁸ zusammen mit R⁷ für Butan-1,4-diyl, Pentan-1,5-diyl oder 3-Oxapentan-1,5-diyl steht.

3. Verbindungen gemäß Anspruch 1 oder 2, **dadurch gekennzeichnet, dass**
R¹ für Wasserstoff oder für jeweils gegebenenfalls durch Fluor substituiertes Methyl, Ethyl, n- oder i-Propyl steht,
R² für Wasserstoff, Cyano, Fluor, Chlor, Brom oder für jeweils gegebenenfalls durch Fluor substituiertes Methyl, Ethyl, n- oder i-Propyl steht,
R⁵ für Wasserstoff oder für einen der Reste -R⁷, -O-R⁷, -S-R⁷, -NH-R⁷ oder -NR⁷R⁸ steht,
R⁷ für jeweils gegebenenfalls einfach oder zweifach substituiertes Methyl, Ethyl, n- oder i-Propyl, n-, i-, s- oder t-Butyl, n-, i-, s-, t- oder neo-Pentyl steht, wobei als Substituenten jeweils vorzugsweise in Betracht kommen:
Cyano, Fluor, Chlor, Brom, Methoxy, Ethoxy, n- oder i-Propoxy, Methoxymethoxy, Ethoxymethoxy, Methoxyethoxy, Ethoxyethoxy, Acetylmethoxy, oder
R⁷ für jeweils gegebenenfalls einfach oder zweifach durch Fluor und/oder Chlor substituiertes Ethenyl, Propenyl, Butenyl, Ethinyl, Propinyl oder Butinyl steht, oder
R⁷ für jeweils gegebenenfalls einfach oder zweifach, gleich oder verschieden durch Fluor, Chlor, Methyl und/oder Ethyl substituiertes Cyclopropyl, Cyclobutyl, Cyclopentyl, Cyclohexyl, Cyclopropylmethyl, Cyclopropylethyl, Cyclopentylmethyl, Cyclopentylethyl, Cyclohexylmethyl oder Cyclohexylethyl steht, oder
R⁷ für jeweils gegebenenfalls einfach, zweifach oder dreifach, gleich oder verschieden substituiertes Phenyl, Benzyl, Phenylethyl, Phenylpropyl, 2,2-Bis-phenyl-ethyl, Phenylethenyl, Phenoxymethyl, Phenoxyethyl, Phenoxypropyl, Furyl, Thienyl, Oxazolyl, Isoxazolyl, Thiazolyl, Pyrazolyl, Oxadiazolyl, Thiadiazolyl, Pyridinyl, Pyrimidinyl, Pyrrolidinyl, Piperidinyl oder Chromanyl steht, wobei als Substituenten jeweils vorzugsweise in Betracht kommen:
Fluor, Chlor, Brom, Cyano, Nitro, Methylendioxy, Methyl, Ethyl, n-oder i-Propyl, n-, i-, s- oder t-Butyl, Methoxy, Ethoxy, n- oder i-Propoxy, n-, Methylthio, Ethylthio, Methylsulfinyl, Methylsulfonyl, Trifluormethyl, Difluormethyl, Trifluormethoxy, Difluormethoxy, und
R⁸ für Wasserstoff oder für jeweils gegebenenfalls einfach substituiertes Methyl, Ethyl, n- oder i-Propyl, n- oder i-Butyl steht, wobei als Substituenten vorzugsweise in Betracht kommen:
Cyano, Fluor, Chlor, Methoxy, Ethoxy, n- oder i-Propoxy, oder
R⁸ für jeweils gegebenenfalls einfach oder zweifach durch Fluor und/oder Chlor - substituiertes Ethenyl, Propenyl, Butenyl, Ethinyl, Propinyl oder Butinyl steht, oder
R⁸ für gegebenenfalls einfach oder zweifach, gleich oder verschieden durch Fluor, Chlor, Methyl und/oder Ethyl substituiertes Cyclopropyl, Cyclopentyl oder Cyclohexyl steht.

4. Verfahren zum Herstellen von Verbindungen gemäß einem der Ansprüche 1 bis 3, **dadurch gekennzeichnet, dass** 2-Aryl-1,2,4-triazin-3,5-di(thi)one der allgemeinen Formel (II) 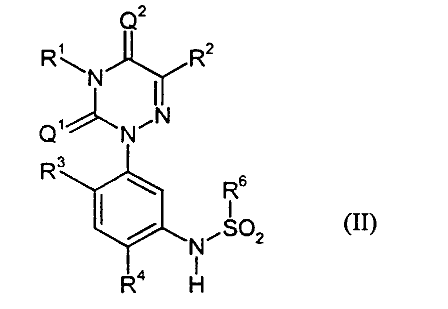 in welcher
Q¹, Q², R¹, R², R³, R⁴ und R⁶ die in einem der Ansprüche 1 bis 3 angegebene Bedeutung haben,
mit Halogencarbonylverbindungen der allgemeinen Formel (III) 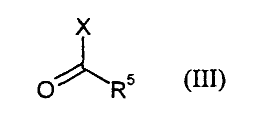 in welcher
R⁵ die oben angegebene Bedeutung hat und
X für Halogen steht,
gegebenenfalls in Gegenwart eines Reaktionshilfsmittels und gegebenenfalls in Gegenwart eines Verdünnungsmittels umgesetzt werden.

5. Verfahren zum Bekämpfen von unerwünschtem Pflanzenwuchs, **dadurch gekennzeichnet, dass** man mindestens eine Verbindung gemäß einem der Ansprüche 1 bis 3 auf die unerwünschten Pflanzen und/oder ihren Lebensraum einwirken lässt.

6. Verwendung von mindestens einer Verbindung gemäß einem der Ansprüche 1 bis 3 zum Bekämpfen von unerwünschten Pflanzen.

7. Herbizide Mittel, **gekennzeichnet durch** einen Gehalt an einer Verbindung gemäß einem der Ansprüche 1 bis 3 und üblichen Streckmitteln und/oder oberflächenaktiven Mitteln.

## Claims

1. A compound of the formula (I) 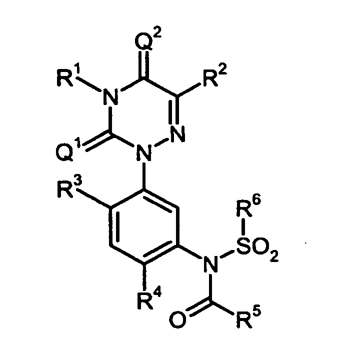 in which
Q¹ represents oxygen or sulfur,
Q² represents oxygen or sulfur,
R¹ represents hydrogen, cyano, amino, represents in each case optionally cyano-, halogen- or C₁-C₄-alkoxy-substituted alkyl, alkoxy, alkylamino, dialkylamino, alkylcarbonyl or alkoxycarbonyl having in each case 1 to 6 carbon atoms in the alkyl groups, represents in each case optionally halogen-substituted alkenyl, alkenylcarbonyl, alkenyloxycarbonyl, alkinyl, alkinylcarbonyl or alkinyloxycarbonyl having in each case 2 to 6 carbon atoms in the alkenyl or alkinyl groups, or represents in each case optionally cyano-, halogen- or C₁-C₄-alkyl-substituted cycloalkyl or cycloalkylalkyl having in each case 3 to 6 carbon atoms in the cycloalkyl groups and optionally 1 to 4 carbon atoms in the alkyl moiety,
R² represents hydrogen, halogen, nitro, carboxyl, cyano, thiocarbamoyl, amino, represents in each case optionally cyano-, halogen- or C₁-C₄-alkoxy-substituted alkyl, alkoxy, alkylthio, alkylamino or dialkylamino having in each case 1 to 6 carbon atoms in the alkyl groups, represents in each case optionally halogen-substituted alkenyl, alkenyloxy, alkenylthio, alkinyl, alkinyloxy or alkinylthio having in each case 2 to 6 carbon atoms in the alkenyl or alkinyl groups, or represents in each case optionally cyano-, halogen- or C₁-C₄-alkyl-substituted cycloalkyl or cycloalkylalkyl having in each case 3 to 6 carbon atoms in the cycloalkyl groups and optionally 1 to 6 carbon atoms in the alkyl moiety,
R³ represents hydrogen, cyano or halogen,
R⁴ represents cyano or thiocarbamoyl,
R⁵ represents hydrogen, represents alkoxycarbonyl having 1 to 6 carbon atoms in the alkoxy group, or represents one of the radicals -R⁷, -O-R⁷, -SR⁷, -NH-R⁷ or -NR⁷R⁸,
R⁶ represents amino, hydroxyl or represents one of the radicals -R⁷ or -NR⁷R⁸,
R⁷ represents straight-chain or branched alkyl having 1 to 10 carbon atoms which is optionally mono- or polysubstituted by identical or different substituents, possible substituents being:
cyano, carboxyl, carbamoyl, thiocarbamoyl, halogen, in each case straight-chain or branched alkoxy, alkoxyalkoxy, alkylcarbonyloxy, alkylthio, alkylsulfinyl, alkylsulfonyl, alkoxycarbonyl, N-alkylaminocarbonyl, N,N-dialkyl-aminocarbonyl, trialkylsilyl or alkylsulfonylaminocarbonyl having in each case 1 to 6 carbon atoms in the individual alkyl moieties, or heterocyclyl, where the heterocyclyl radical is a five- to seven-membered, optionally benzo-fused, saturated or unsaturated heterocycle having 1 to 3 identical or different-heteroatoms, in particular nitrogen, oxygen and/ or sulfur, or
R⁷ represents allcenyl or alkinyl having in each case 2 to 8 carbon atoms and being in each case optionally mono- or polysubstituted by identical or different halogens, or
R⁷ represents cycloalkyl or cycloalkylalkyl having in each case 3 to 7 carbon atoms in the cycloalkyl groups and optionally 1 to 4 carbon atoms in the alkyl moiety and being in each case optionally mono- or polysubstituted by identical or different substituents from the group consisting of cyano, halogen and C₁-C₄-alkyl, or
R⁷ represents aryl, bisarylalkyl, arylalkenyl, arylalkyl, aryloxyalkyl or arylalkoxyaucyl having in each case 6 to 10 carbon atoms in the aryl moiety and optionally up to 4 carbon atoms in the straight-chain or branched alkyl moiety or alkenyl moiety and being in each case optionally mono- or polysubstituted in the aryl moiety by identical or different substituents, or represents a saturated or unsaturated five- to seven-membered heterocyclyl radical having 1 to 3 identical or different heteroatoms - in particular nitrogen, oxygen and/or sulfur - and being optionally mono- or polysubstituted by identical or different substituents and/or benzo-fused, possible aryl or heterocyclyl substituents being:
halogen, cyano, nitro, amino, methylenedioxy, N-(C₁-C₄-alkylcarbonyl)-amino, in each case straight-chain or branched alkyl, alkoxy, alkylthio, alkylsulfinyl or alkylsulfonyl having in each case 1 to 6 carbon atoms, in each case straight-chain or branched halogenoalkyl, halogenoalkoxy, halogenoalkylthio, halogenoalkylsulfinyl or halogenoalkylsulfonyl having in each case 1 to 6 carbon atoms and 1 to 3 identical or different halogen atoms, in each case straight-chain or branched alkoxycarbonyl or alkoximinoalkyl having in each case 1 to 6 carbon atoms in the individual alkyl moieties and phenyl which is optionally mono- or polysubstituted by identical or different substituents from the group consisting of halogen and straight-chain or branched alkyl or alkoxy having in each case 1 to 6 carbon atoms and straight-chain or branched halogenoalkyl or halogenoallcoxy having in each case 1 to 6 carbon atoms and 1 to 3 identical or different halogen atoms, and
R⁸ represents hydrogen or represents straight-chain or branched alkyl having 1 to 8 carbon atoms which is optionally mono- or polysubstituted by identical or different substituents, possible substituents being:
cyano, carboxyl, carbamoyl, thiocarbamoyl, halogen, in each case straight-chain or branched alkoxy, alkoxyalkoxy, alkylthio, alkylsulfinyl, alkylsulfonyl, alkoxycarbonyl, alkylaminocarbonyl, dialkylaminocarbonyl, triallcylsilyl or alkylsulfonylaminocarbonyl having in each case 1 to 8 carbon atoms in the individual alkyl moieties or heterocyclyl, where the heterocyclyl radical is a five- to seven-membered, optionally benzo-fused, saturated or unsaturated heterocycle having 1 to 3 identical or different heteroatoms, in particular nitrogen, oxygen and/or sulfur, or
R⁸ represents alkenyl or alkinyl having in each case 2 to 8 carbon atoms and being in each case optionally mono- or polysubstituted by identical or different halogens, or
R⁸ represents cycloalkyl having 3 to 7 carbon atoms which is optionally mono- or polysubstituted by identical or different substituents from the group consisting of cyano, halogen and straight-chain or branched alkyl having 1 to 4 carbon atoms, or
R⁸ together with R⁷ represents alkanediyl (alkylene) having 2 to 6 carbon atoms which is optionally interrupted by O (oxygen), S (sulfur), NH or N-(C₁-C₄-alkyl).

2. A compound as claimed in claim 1, wherein
R¹ represents hydrogen, cyano, amino, represents in each case optionally cyano-, fluorine-, chlorine-, methoxy- or ethoxy-substituted methyl, ethyl, n- or i-propyl, n-, i-, s- or t-butyl, methoxy, ethoxy, n- or i-propoxy, n-, i-, s- or t-butoxy, methylamino, ethylamino, n- or i-propylamino, n-, i-, s- or t-butylamino, dimethylamino, diethylamino, acetyl, propionyl, n- or i-butyroyl, methoxycarbonyl, ethoxycarbonyl, n- or i-propoxycarbonyl, represents in each case optionally fluorine-, chlorine- or bromine-substituted propenyl, butenyl, propenylcarbonyl, butenylcarbonyl, propenyloxycarbonyl, butenyloxycarbonyl, represents propinyl, butinyl, propinylcarbonyl, butinylcarbonyl, propinyloxycarbonyl or butinyloxycarbonyl, or represents in each case optionally cyano-, fluorine-, chlorine-, bromine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl,
R² represents hydrogen, nitro, carboxyl, cyano, thiocarbamoyl, amino, fluorine, chlorine, bromine, represents in each case optionally cyano-, fluorine-, chlorine-, methoxy- or ethoxy-substituted methyl, ethyl, n- or i-propyl, n-, i- s- or t-butyl, methoxy, ethoxy, n- or i-propoxy, n-, i-, s- or t-butoxy, methylthio, ethylthio, n- or i-propylthio, n-, i-, s- or t-butylthio, methylamino, ethylamino, n- or i-propylamino, n-, i-, s- or t-butylamino, dimethylamino or diethylamino, represents in each case optionally fluorine-, chlorine- or bromine-substituted propenyl, propenyloxy, propenylthio, butenyl, butenyloxy or butenylthio, represents propinyl, propinyloxy, propinylthio, butinyl, butinyloxy or butinylthio, or represents in each case optionally cyano-, fluorine-, chlorine-, bromine-, methyl- or ethyl-substituted cyclopropyl, cyclobutyl, cyclopentyl, cyclohexyl, cyclopropylmethyl, cyclobutylmethyl, cyclopentylmethyl or cyclohexylmethyl,
R³ represents hydrogen, fluorine or chlorine,
R⁵ represents hydrogen, methoxycarbonyl, ethoxycarbonyl, n- or i-propoxycarbonyl, or represents one of the radicals -R⁷, -O-R⁷, -S-R⁷, -NH-R⁷ or -NR⁷R⁸,
R⁷ represents methyl, ethyl, n- or i-propyl, n-, i-, s- or t-butyl, n-, i-, s-, t- or neo-pentyl, n-, i- or s-hexyl, each of which is optionally mono- or disubstituted, preferred possible substituents being in each case: cyano, carboxyl, carbamoyl, fluorine, chlorine, bromine, methoxy, ethoxy, n- or i-propoxy, methoxymethoxy, ethoxymethoxy, methoxyethoxy, ethoxyethoxy, acetylmethoxy, methylthio, ethylthio, n- or i-propylthio, methylsulfinyl, ethylsulfinyl, methylsulfonyl, ethylsulfonyl, methoxycarbonyl, ethoxycarbonyl, methylaminocarbonyl, ethyl-aminocarbonyl, dimethylaminocarbonyl, trimethylsilyl, methylsulfonylaminocarbonyl or ethylsulfonylaminocarbonyl, or
R⁷ represents ethenyl, propenyl, butenyl, ethinyl, propinyl or butinyl, each of which is optionally mono- or disubstituted by fluorine and/or chlorine, or
R⁷ represents cyclopropyl, cyclobutyl, cyclopentyl, cyclohexyl, cyclopropylmethyl, cyclopropylethyl, cyclopentylmethyl, cyclopentylethyl, cyclohexylmethyl or cyclohexylethyl, each of which is optionally mono- or disubstituted by identical or different substituents from the group consisting of fluorine, chlorine, methyl and ethyl, or
R⁷ represents phenyl, benzyl, phenylethyl, phenylpropyl, phenoxymethyl, 2,2-bisphenylethyl, phenylethenyl, phenoxyethyl, phenoxypropyl, furyl, furylmethyl, thienyl, oxazolyl, isoxazolyl, thiazolyl, pyrazolyl, oxadiazolyl, thiadiazolyl, pyridinyl, pyridinylmethyl, pyrimidinyl, pyrrolidinyl, piperidinyl, morpholinyl or chromanyl, each of which is optionally mono-, di- or trisubstituted by identical or different substituents, preferred possible substituents being in each case:
fluorine, chlorine, bromine, cyano, nitro, amino, methylenedioxy, N-acetylamino, methyl, ethyl, n- or i-propyl, n-, i-, s- or t-butyl, methoxy, ethoxy, n- or i-propoxy, n-, i-, s- or t-butoxy, methylthio, ethylthio, methylsulfinyl, methylsulfonyl, trifluoromethyl, difluoromethyl, trifluoromethoxy, difluoromethoxy, trifluoromethylthio, trifluoromethylsulfmyl, trifluoromethylsulfonyl, methoxycarbonyl, ethoxycarbonyl, methoximinomethyl, methoximinoethyl, ethoximinomethyl, ethoximinoethyl, and
R⁸ represents hydrogen or represents in each case optionally mono-substituted methyl, ethyl, n- or i-propyl, n- or i-butyl, preferred possible substituents being:
cyano, carboxyl, carbamoyl, fluorine, chlorine, methoxy, ethoxy, n- or i-propoxy, methoxymethoxy, ethoxymethoxy, methoxyethoxy, ethoxyethoxy, methylthio, ethylthio, n- or i-propylthio, methylsulfinyl, ethylsulfinyl, methylsulfonyl, ethylsulfonyl, methoxycarbonyl, ethoxycarbonyl, methylaminocarbonyl, ethylaminocarbonyl, dimethylaminocarbonyl, or
R⁸ represents ethenyl, propenyl, butenyl, ethinyl, propinyl or butinyl, each of which is optionally mono- or disubstituted by fluorine and/or chlorine, or
R⁸ represents cyclopropyl, cyclopentyl or cyclohexyl, each of which is optionally mono- or disubstituted by identical or different substituents from the group consisting of fluorine, chlorine, methyl and ethyl, or
R⁸ together with R⁷ represents butane-1,4-diyl, pentane-1,5-diyl or 3-oxa-pentane-1,5-diyl.

3. A compound as claimed in claim 1 or 2, wherein
R¹ represents hydrogen or represents in each case optionally fluorine-substituted methyl, ethyl, n- or i-propyl,
R² represents hydrogen, cyano, fluorine, chlorine, bromine or represents in each case optionally fluorine-substituted methyl, ethyl, n- or i-propyl,
R⁵ represents hydrogen or represents one of the radicals -R⁷, -O-R⁷, -S-R⁷, -NH-R⁷ or -NR⁷R⁸,
R⁷ represents in each case optionally mono- or disubstituted methyl, ethyl, n- or i-propyl, n-, i-, s- or t-butyl, n-, i-, s-, t- or neo-pentyl, preferred possible substituents being in each case:
cyano, fluorine, chlorine, bromine, methoxy, ethoxy, n- or i-propoxy, methoxymethoxy, ethoxymethoxy, methoxyethoxy, ethoxyethoxy, acetylmethoxy, or
R⁷ represents ethenyl, propenyl, butenyl, ethinyl, propinyl or butinyl, each of which is optionally mono- or disubstituted by fluorine and/or chlorine, or
R⁷ represents cyclopropyl, cyclobutyl, cyclopentyl, cyclohexyl, cyclopropylmethyl, cyclopropylethyl, cyclopentylmethyl, cyclopentylethyl, cyclohexylmethyl or cyclohexylethyl, each of which is optionally mono- or disubstituted by identical or different substituents from the group consisting of fluorine, chlorine, methyl and ethyl, or
R⁷ represents phenyl, benzyl, phenylethyl, phenylpropyl, 2,2-bisphenylethyl, phenylethenyl, phenoxymethyl, phenoxyethyl, phenoxypropyl, furyl, thienyl, oxazolyl, isoxazolyl, thiazolyl, pyrazolyl, oxadiazolyl, thiadiazolyl, pyridinyl, pyrimidinyl, pyrrolidinyl, piperidinyl or chromanyl, each of which is optionally mono-, di- or trisubstituted by identical or different substituents, preferred possible substituents being in each case:
fluorine, chlorine, bromine, cyano, nitro, methylenedioxy, methyl, ethyl, n- or i-propyl, n-, i-, s- or t-butyl, methoxy, ethoxy, n- or i-propoxy, methylthio, ethylthio, methylsulfinyl, methylsulfonyl, trifluoromethyl, difluoromethyl, trifluoromethoxy, difluoromethoxy, and
R⁸ represents hydrogen or represents in each case optionally monosubstituted methyl, ethyl, n- or i-propyl, n- or i-butyl, preferred possible substituents being:
cyano, fluorine, chlorine, methoxy, ethoxy, n- or i-propoxy, or
R⁸ represents ethenyl, propenyl, butenyl, ethinyl, propinyl or butinyl, each of which is optionally mono- or disubstituted by fluorine and/or chlorine, or
R⁸ represents cyclopropyl, cyclopentyl or cyclohexyl, each of which is optionally mono- or disubstituted by identical or different substituents from the group consisting of fluorine, chlorine, methyl and ethyl.

4. A process for preparing compounds as claimed in any of claims 1 to 3, wherein 2-aryl-1,2,4-triazine-3,5-di(thi)ones of the formula (II) 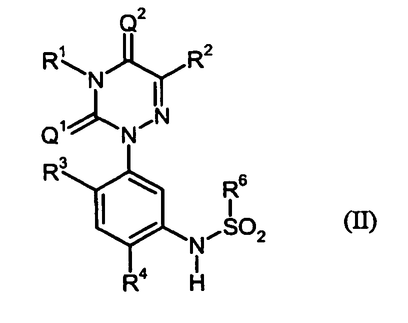 in which
Q¹, Q², R¹, R², R³, R⁴ and R⁶ are each as defined in any of Claims 1 to 3
are reacted with halogenocarbonyl compounds of the general formula (III) 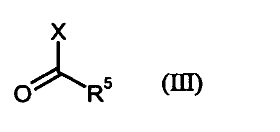 in which
R⁵ is as defined above and
X represents halogen,
if appropriate in the presence of a reaction auxiliary and if appropriate in the presence of a diluent.

5. A method for controlling undesirable vegetation, wherein at least one compound as claimed in any of claims 1 to 3 is allowed to act on the undesirable plants and/or their habitat.

6. The use of at least one compound as claimed in any of claims 1 to 3 for controlling undesirable plants.

7. A herbicidal composition, which comprises a compound as claimed in any of claims 1 to 3 and customary extenders and/or surfactants.

## Revendications

1. Composés de formule générale (I) 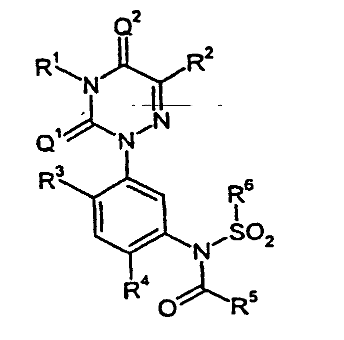 dans laquelle
Q¹ représente un atome d'oxygène ou de soufre,
Q² représente un atome d'oxygène ou de soufre,
R¹ représente un atome d'hydrogène, un groupe cyano, amino, un groupe alkyle, alkoxy, alkylamino, dialkylamino, alkylcarbonyle ou alkoxycarbonyle, à chaque fois présentant 1 à 6 atomes de carbone dans les groupes alkyle, à chaque fois éventuellement substitué par un substituant cyano, halogène ou alkoxy en C₁-C₄, un groupe alcényle, alcénylcarbonyle, alcényloxy-carbonyle, alcynyle, alcynylcarbonyle ou alcynyloxycarbonyle, à chaque fois présentant 2 à 6 atomes de carbone dans les groupes alcényle ou alcynyle, à chaque fois éventuellement substitué par un substituant halogène, ou un groupe cycloalkyle
ou cycloalkylalkyle, à chaque fois présentant 3 à 6 atomes de carbone dans les groupes cycloalkyle et éventuellement 1 à 4 atomes de carbone dans le fragment alkyle, à chaque fois éventuellement substitué par un substituant cyano, halogène ou alkyle en C₁-C₄,
R² représente un atome d'hydrogène, un atome d'halogène, un groupe nitro, carboxy, cyano, thiocarbamoyle, amino, un groupe alkyle, alkoxy, alkylthio, alkylamino ou dialkylamino, à chaque fois présentant 1 à 6 atomes de carbone dans les groupes alkyle, à chaque fois éventuellement substitué par un substituant cyano, halogène ou alkoxy en C₁-C₄, un groupe alcényle, alcényloxy, alcénylthio, alcynyle, alcynyloxy ou alcynylthio, à chaque fois présentant 2 à 6 atomes de carbone dans les groupes alcényle ou alcynyle, à chaque fois éventuellement substitué par un atome d'halogène, ou un groupe cycloalkyle ou cycloalkylalkyle, à chaque fois présentant 3 à 6 atomes de carbone dans les groupes cycloalkyle et éventuellement 1 à 6 atomes de carbone dans le fragment alkyle, à chaque fois éventuellement substitué par un substituant cyano, halogène ou alkyle en C₁-C₄,
R³ représente un atome d'hydrogène, un groupe cyano ou halogène,
R⁴ représente un groupe cyano ou thiocarbamoyle,
R⁵ représente un atome d'hydrogène, un groupe alkoxycarbonyle présentant 1 à 6 atomes de carbone dans le groupe alkoxy, ou représente un des restes -R⁷, -O-R⁷, -SR⁷, -NH-R⁷ ou -NR⁷R⁸,
R⁶ représente un groupe amino, hydroxy ou un des restes -R⁷ ou -NR⁷R⁸,
R⁷ représente un groupe alkyle linéaire ou ramifié, présentant 1 à 10 atomes de carbone, éventuellement substitué une ou plusieurs fois par des substituants identiques ou différents, en tant que substituants entrent en ligne de compte :
cyano, carboxy, carbamoyle, thiocarbamoyle, halogène, alkoxy, alkoxyalkoxy, alkylcarbonyloxy, alkylthio, alkylsulfinyle, alkylsulfonyle, alkoxycarbonyle, N-alkylaminocarbonyle, N,N-dialkyl-aminocarbonyle, trialkylsilyle ou alkylsulfonylaminocarbonyle, à chaque fois linéaire ou ramifié, à chaque fois présentant 1 à 6 atomes de carbone dans les différents fragments alkyle, ou hétérocyclyle, le reste hétérocyclyle étant un reste de 5 à 7 chaînons, saturé ou insaturé, éventuellement condensé sur un cycle benzène, présentant 1 à 3 hétéroatomes identiques ou différents, en particulier l'azote, l'oxygène et/ou le soufre, ou
R⁷ représente un groupe alcényle ou alcynyle, à chaque fois présentant 2 à 8 atomes de carbone, à chaque fois éventuellement substitué par un
ou plusieurs substituants halogène identiques
ou différents, ou
R⁷ représente un groupe cycloalkyle ou cycloalkylalkyle, à chaque fois présentant 3 à 7 atomes de carbone dans les groupes cycloalkyle et éventuellement 1 à 4 atomes de carbone dans le fragment alkyle, à chaque fois éventuellement substitué une ou plusieurs fois par des substituants identiques ou différents cyano, halogène et/ou alkyle en C₁-C₄, ou
R⁷ représente un groupe aryle, bis-arylalkyle, arylalcényle, arylalkyle, aryloxyalkyle ou arylalkoxyalkyle, à chaque fois présentant 6 à 10 atomes de carbone dans le fragment aryle et éventuellement jusqu'à 4 atomes de carbone dans le fragment alkyle ou alcényle linéaire ou ramifié, à chaque fois éventuellement substitué dans le fragment aryle une ou plusieurs fois par des substituants identiques ou différents,
ou représente un reste hétérocyclyle de 5 à 7 chaînons, saturé ou insaturé, éventuellement substitué une ou plusieurs fois par des substituants identiques ou différents et/ou condensé sur un cycle benzène, présentant 1 à 3 hétéroatomes identiques ou différents, en particulier un atome d'azote, d'oxygène et/ou de soufre, en tant que substituants des groupes aryle ou hétérocyclyle entrent en ligne de compte :
halogène, cyano, nitro, amino, méthylènedioxy, N-[(alkyl en C₁-C₄)-carbonyl]-amino, un groupe alkyle, alkoxy, alkylthio, alkylsulfinyle ou alkylsulfonyle, à chaque fois linéaire ou ramifié, à chaque fois présentant 1 à 6 atomes de carbone, un groupe halogénoalkyle, halogénoalkoxy, halogénoalkylthio, halogénoalkylsulfinyle ou halogénoalkylsulfonyle, à chaque fois linéaire ou ramifié, à chaque fois présentant 1 à 6 atomes de carbone et 1 à 3 atomes d'halogènes identiques ou différents, un groupe alkoxycarbonyle ou alkoxyiminoalkyle, à chaque fois linéaire ou ramifié, à chaque fois présentant 1 à 6 atomes de carbone dans les différents fragments alkyle, ainsi qu'éventuellement une ou plusieurs fois par des substituants identiques ou différents halogène et/ou alkyle ou alkoxy linéaire ou ramifié, à chaque fois présentant 1 à 6 atomes de carbone, et/ou halogénoalkyle ou halogénoalkoxy linéaire ou ramifié, à chaque fois présentant 1 à 6 atomes de carbone, et un groupe phényle substitué par 1 à 3 atomes d'halogènes identiques ou différents, et
R⁸ représente un atome d'hydrogène ou un groupe alkyle linéaire ou ramifié, présentant 1 à 8 atomes de carbone, éventuellement substitué une ou plusieurs fois par des substituants identiques ou différents, en tant que substituants entrent en ligne de compte :
cyano, carboxy, carbamoyle, thiocarbamoyle, halogène, alkoxy, alkoxyalkoxy, alkylthio, alkylsulfinyle, alkylsulfonyle, alkoxycarbonyle, alkylaminocarbonyle, dialkylaminocarbonyle, trialkylsilyle ou alkylsulfonylaminocarbonyle, à chaque fois linéaire ou ramifié, à chaque fois présentant 1 à 8 atomes de carbone dans les différents fragments alkyle, ou hétérocyclyle, le reste hétérocyclyle est un hétérocycle de 5 à 7 chaînons, saturé ou insaturé, éventuellement condensé sur le cycle benzène, présentant 1 à 3 hétéroatomes identiques ou différents, en particulier un atome d'azote, d'oxygène et/ou de soufre, ou
R⁸ représente un groupe alcényle ou alcynyle, à chaque fois présentant 2 à 8 atomes de carbone, à chaque fois éventuellement substitué une ou plusieurs fois par des substituants halogènes identiques ou différents, ou
R⁸ représente un groupe cycloalkyle présentant 3 à 7 atomes de carbone, éventuellement substitué une ou plusieurs fois par des substituants identiques ou différents cyano, halogène et/ou alkyle linéaire ou ramifié présentant 1 à 4 atomes de carbone, ou
R⁸ conjointement avec R⁷ représentent un groupe alcanediyle (alkylène) présentant 2 à 6 atomes de carbone, éventuellement interrompu par O (oxygène), S (soufre), NH ou N (alkyle en C₁-C₄) .

2. Composés selon la revendication 1,
**caractérisés en ce que**
R¹ représente un atome d'hydrogène, un groupe cyano, amino, un groupe méthyle, éthyle, n- ou i-propyle, n-, i-, s- ou t-butyle, méthoxy, éthoxy, n- ou i-propoxy, n-, i-, s- ou t- butoxy, méthylamino, éthylamino, n- ou i- propylamino, n-, i-, s- ou t-butylamino, diméthylamino, diéthylamino, acétyle, propionyle, n- ou i-butyroyle, méthoxycarbonyle, éthoxycarbonyle, n- ou i-propoxycarbonyle, à chaque fois éventuellement substitué par un substituant cyano, fluor, chlore, méthoxy ou éthoxy, un groupe propényle, butényle, propénylcarbonyle, buténylcarbonyle, propényloxycarbonyle, butényloxycarbonyle, un groupe propynyle, butynyle, propynylcarbonyle, butynylcarbonyle, propynyloxycarbonyle ou butynyloxycarbonyle, à chaque fois éventuellement substitué par un substituant fluor, chlore ou brome, ou un groupe cyclopropyle, cyclobutyle, cyclopentyle, cyclohexyle, cyclopropylméthyle, cyclobutylméthyle, cyclopentylméthyle ou cyclohexylméthyle, à chaque fois éventuellement substitué par un substituant cyano, fluor, chlore, brome, méthyle ou éthyle,
R² représente un atome d'hydrogène, un groupe nitro, carboxy, cyano, thiocarbamoyle, amino, fluor, chlore, brome, un groupe méthyle, éthyle, n- ou i-propyle, n-, i- s- ou t-butyle, méthoxy, éthoxy, n- ou i-propoxy, n-, i-, s- ou t-butoxy, méthylthio, éthylthio, n- ou i- propylthio, n-, i-, s- ou t-butylthio, méthylamino, éthylamino, n- ou i-propylamino, n-, i-, s- ou t-butylamino, diméthylamino ou diéthylamino, à chaque fois éventuellement substitué par un substituant cyano, fluor, chlore, méthoxy ou éthoxy, un groupe propényle, propényloxy, propénylthio, butényle, butényloxy ou buténylthio, un groupe propynyle, propynyloxy, propynylthio, butynyle, butynyloxy ou butynylthio, à chaque fois éventuellement substitué par un substituant fluor, chlore ou brome, ou un groupe cyclopropyle, cyclobutyle, cyclopentyle, cyclohexyle, cyclopropylméthyle, cyclobutylméthyle, cyclopentylméthyle ou cyclohexylméthyle, à chaque fois éventuellement substitué par un substituant cyano, fluor, chlore, brome, méthyle ou éthyle,
R³ représente un atome d'hydrogène, de fluor ou de chlore,
R⁵ représente un atome d'hydrogène, un groupe méthoxycarbonyle, éthoxycarbonyle, n- ou i- propoxycarbonyle ou un des restes -R⁷, -O-R⁷, -S-R⁷, -NH-R⁷ ou -NR⁷R⁸,
R⁷ représente un groupe méthyle, éthyle, n- ou i- propyle, n-, i-, s- ou t-butyle, n-, i-, s-, t-
ou néo-pentyle, n-, i- ou s-hexyle, à chaque fois éventuellement substitué une ou deux fois, en tant que substituants entrent en ligne de compte de préférence :
cyano, carboxy, carbamoyle, fluor, chlore, brome, méthoxy, éthoxy, n- ou i-propoxy, méthoxyméthoxy, éthoxyméthoxy, méthoxyéthoxy, éthoxyéthoxy, acétylméthoxy, méthylthio, éthylthio, n- ou i-propylthio, méthylsulfinyle, éthylsulfinyle, méthylsulfonyle, éthylsulfonyle, méthoxycarbonyle, éthoxycarbonyle, méthylaminocarbonyle, éthylaminocarbonyle, diméthylaminocarbonyle, triméthylsilyle, méthylsulfonylaminocarbonyle ou éthylsulfonylaminocarbonyle, ou
R⁷ représente un groupe éthényle, propényle, butényle, éthynyle, propynyle ou butynyle, à chaque fois éventuellement substitué une ou deux fois par un substituant fluor et/ou chlore, ou
R⁷ représente un groupe cyclopropyle, cyclobutyle, cyclopentyle, cyclohexyle, cyclopropylméthyle, cyclopropyléthyle, cyclopentylméthyle, cyclopentyléthyle, cyclohexylméthyle ou cyclohexyléthyle, à chaque fois éventuellement substitué une ou deux fois par des substituants identiques ou différents fluor, chlore, méthyle et/ou éthyle, ou
R⁷ représente un groupe phényle, benzyle, phényléthyle, phénylpropyle, phénoxyméthyle, 2,2-bis-phényl-éthyle, phényléthényle, phénoxyéthyle, phénoxypropyle, furyle, furylméthyle, thiényle, oxazolyle, isoxazolyle, thiazolyle, pyrazolyle, oxadiazolyle, thiadiazolyle, pyridynyle, pyridynylméthyle, pyrimidynyle, pyrrolidynyle, pipéridynyle, morpholinyle ou chromanyle, à chaque fois éventuellement substitué une, deux ou trois fois par des substituants identiques ou différents, en tant que substituants entrent en ligne de compte de préférence :
fluor, chlore, brome, cyano, nitro, amino, méthylènedioxy, N-acétylamino, méthyle, éthyle, n- ou i-propyle, n-, i-, s- ou t-butyle, méthoxy, éthoxy, n- ou i-propoxy, n-, i-, s- ou t-butoxy, méthylthio, éthylthio, méthylsulfinyle, méthylsulfonyle, trifluorométhyle, difluorométhyle, trifluorométhoxy, difluorométhoxy, trifluorométhylthio, trifluorométhylsulfinyle, trifluorométhylsulfonyle, méthoxycarbonyle, éthoxycarbonyle, méthoxyiminométhyle, méthoxyiminoéthyle, éthoxyiminométhyle, éthoxyiminoéthyle, et
R⁸ représente un atome d'hydrogène ou un groupe méthyle, éthyle, n- ou i-propyle, n- ou i- butyle, à chaque fois éventuellement substitué une fois, en tant que substituants entrent en ligne de compte de préférence :
cyano, carboxyle, carbamoyle, fluor, chlore, méthoxy, éthoxy, n- ou i-propoxy, méthoxyméthoxy, éthoxyméthoxy, méthoxyéthoxy, éthoxyéthoxy, méthylthio, éthylthio, n- ou i- propylthio, méthylsulfinyle, éthylsulfinyle, méthylsulfonyle, éthylsulfonyle, méthoxycarbonyle, éthoxycarbonyle, méthylaminocarbonyle, éthylaminocarbonyle, diméthylaminocarbonyle, ou
R⁸ représente un groupe éthényle, propényle, butényle, éthynyle, propynyle ou butynyle, à chaque fois éventuellement substitué une ou deux fois par un substituant fluor et/ou chlore, ou
R⁸ représente un groupe cyclopropyle, cyclopentyle ou cyclohexyle, éventuellement substitué une ou deux fois par des substituants identiques ou différents fluor, chlore, méthyle et/ou éthyle, ou
R⁸ conjointement avec R⁷ représentent un groupe butane-1,4-diyle, pentane-1,5-diyle ou 3-oxapentane-1,5-diyle.

3. Composés selon la revendication 1 ou 2, **caractérisés en ce que**
R¹ représente un atome d'hydrogène ou un groupe méthyle, éthyle, n- ou i-propyle, à chaque fois éventuellement substitué par un substituant fluor,
R² représente un atome d'hydrogène, un groupe cyano, fluor, chlore, brome, ou un groupe méthyle, éthyle, n- ou i-propyle, à chaque fois éventuellement substitué par un substituant fluor,
R⁵ représente un atome d'hydrogène ou un des restes -R⁷, -O-R⁷, -S-R⁷, -NH-R⁷ ou -NR⁷R⁸,
R⁷ représente un groupe méthyle, éthyle, n- ou i- propyle, n-, i-, s- ou t-butyle, n-, i-, s-, t- ou néo-pentyle, à chaque fois éventuellement substitué une ou deux fois, en tant que substituants entrent en ligne de compte : cyano, fluor, chlore, brome, méthoxy, éthoxy, n- ou i-propoxy, méthoxyméthoxy, éthoxyméthoxy, méthoxyéthoxy, éthoxyéthoxy, acétylméthoxy, ou
R⁷ représente un groupe éthényle, propényle, butényle, éthynyle, propynyle ou butynyle, à chaque fois éventuellement substitué une ou deux fois par un substituant fluor et/ou chlore, ou
R⁷ représente un groupe cyclopropyle, cyclobutyle, cyclopentyle, cyclohexyle, cyclopropylméthyle, cyclopropyléthyle, cyclopentylméthyle, cyclopentyléthyle, cyclohexylméthyle ou cyclohexyléthyle, à chaque fois éventuellement substitué une ou deux fois par des substituants identiques ou différents fluor, chlore, méthyle et/ou éthyle, ou
R⁷ représente un groupe phényle, benzyle, phényléthyle, phénylpropyle, 2,2-bis-phényléthyle, phényléthényle, phénoxyméthyle, phénoxyéthyle, phénoxypropyle, furyle, thiényle, oxazolyle, isoxazolyle, thiazolyle, pyrazolyle, oxadiazolyle, thiadiazolyle, pyridynyle, pyrimidynyle, pyrrolidynyle, pipéridynyle ou chromanyle, à chaque fois éventuellement substitué une, deux ou trois fois par des substituants identiques ou différents, en tant que substituants entrent en ligne de compte de préférence :
fluor, chlore, brome, cyano, nitro, méthylènedioxy, méthyle, éthyle, n- ou i- propyle, n-, i-, s- ou t-butyle, méthoxy, éthoxy, n- ou i-propoxy, n-, méthylthio, éthylthio, méthylsulfinyle, méthylsulfonyle, trifluorométhyle, difluorométhyle, trifluorométhoxy, difluorométhoxy, et
R⁸ représente un atome d'hydrogène ou un groupe méthyle, éthyle, n- ou i-propyle, n- ou i - butyle, à chaque fois éventuellement substitué une fois, en tant que substituants entrent en ligne de compte de préférence : cyano, fluor, chlore, méthoxy, éthoxy, n- ou i- propoxy, ou
R⁸ représente un groupe éthényle, propényle, butényle, éthynyle, propynyle ou butynyle, à chaque fois éventuellement substitué une ou deux fois par un substituant fluor et/ou chlore, ou
R⁸ représente un groupe cyclopropyle, cyclopentyle
ou cyclohexyle, à chaque fois éventuellement substitué une ou deux fois par des substituants identiques ou différents fluor, chlore, méthyle et/ou éthyle.

4. Procédé pour la préparation de composés selon l'une des revendications 1 à 3, **caractérisé en ce qu'**on fait réagir une 2-aryl-1,2,4-triazine-3,5-di(thi)one de formule générale (II) 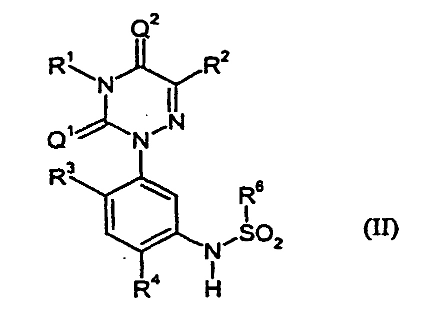 dans laquelle
Q¹, Q², R¹, R², R³, R⁴ et R⁶ possèdent la signification
donnée dans l'une des revendications 1 à 3,
sur un composé de halogénocarbonyle de formule générale (III) 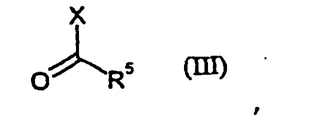 dans laquelle
R⁵ possède la signification donnée ci-dessus et
X représente un atome d'halogène,
éventuellement en présence d'un agent auxiliaire de réaction et éventuellement en présence d'un diluant.

5. Procédé pour la lutte contre la croissance d'une végétation indésirable, **caractérisé en ce qu'**on laisse agir au moins un composé selon l'une des revendications 1 à 3 sur des plantes indésirables et/ou leur environnement.

6. Utilisation d'au moins un composé selon l'une des revendications 1 à 3 pour la lutte contre des plantes indésirables.

7. Agents herbicides **caractérisés par** une teneur en un composé selon l'une des revendications 1 à 3 et des agents extenseurs et/ou tensio-actifs usuels.
